# EUROPEAN PATENT SPECIFICATION

(11) **EP 3 325 024 B1**
(45) Date of publication and mention of the grant of the patent: **23.12.2020**
(21) Application number: 16738333.0
(22) Date of filing: 29.06.2016
(51) Int. Cl.: A61L 15/36, A61F 13/00, A61L 15/44, A61L 15/46

(54) **PATCH CONTAINING MICROORGANISM**
PATCH-HALTIGER MIKROORGANISMUS
PATCH CONTENANT UN MICROORGANISME

(30) Priority: 23.07.2015 US 201562196258 P
(43) Date of publication of application: 30.05.2018
(73) Proprietor: The Procter & Gamble Company, Cincinnati, OH 45202 (US)
(72) Inventor: AVILES, Misael, Omar, Cincinnati, OH 45202 (US); CASERTA, Justin, Angelo, Cincinnati, OH 45202 (US); DUVAL, Dean, Larry, Cincinnati, OH 45202 (US); ELLINGSON, Peter, Christopher, Cincinnati, OH 45202 (US); KEEGAN, Sharon, Anne, Cincinnati, OH 45202 (US); MCKIERNAN, Robin, Lynn, Cincinnati, OH 45202 (US); YOUNG, Roger, Dale, Cincinnati, OH 45202 (US); SONG, Brian, Xiaoqing, Cincinnati, OH 45202 (US)
(74) Representative: P&G Patent Germany
(86) International application number: PCT/US2016/039933
(87) International publication number: WO 2017/014929

(56) References cited:
- EP-A1- 1 118 339
- WO-A1-2004/105822
- WO-A1-2012/003365
- WO-A1-2015/112374
- US-A- 5 569 230
- US-A1- 2004 243 076

## Description

### FIELD OF THE INVENTION

The invention relates to patches comprising a fibrous element comprising a filament-forming material and a microorganism.

### BACKGROUND OF THE INVENTION

Infections of the urogenital tract such as bacterial vaginosis, urinary tract infection and yeast infections in women represent a major burden on the quality of life of women. Treatment most often is based on antibacterial and/or antifungal therapies using antibacterial and/or antifungal agents. However, though such antibacterial/antifungal therapies may work, there is a high level of recurrence. Treatments are often limited to repeated courses of antimicrobial therapy to treat recurrence and they may also in themselves cause a disturbed microflora. In addition, beyond treating infections, women are interested in improving vaginal health to reduce vaginal odor and improve cleanliness.

Administration of probiotics to the urogenital area or the skin has been suggested as one approach to outcompete pathogenic species and facilitate reestablishment and maintenance of a beneficial microbial flora and balance in these areas, and to deliver on health and cleanliness and reducing odor. By incorporating the probiotics in an absorbent article, like a sanitary pad, a pantiliner or a tampon the consumer can passively wear the article like she does today and benefit by the delivery of the probiotic into the vaginal area. WO 2010/74614 discloses a sanitary article comprising a closed chamber type delivery member made of a metal foil, a polymeric film or a laminate for delivery of an additive such as a skin caring agent and probiotics. It further discloses probiotic bacteria are preferably delivered in a hydrophobic carrier to prolong the survival of the bacteria during manufacture, transport, and storage though it provides no working example of probiotic bacteria. WO2000/61201 discloses a sanitary article containing acid-producing bacteria in a spore form useful for inhibiting growth of parasites and pathogens on the epithelial tissue, wherein the bacteria is incorporated into the absorbent product as a liquid, paste, power, granule, or pellet formulation. EP 1 118 339 discloses breathable absorbent articles, particularly sanitary napkins and panty liners, which comprise spores which in their living form exhibit antagonistic properties against undesirable strains of microorganisms. WO-A-2004-105822 discloses film-shaped polymer matrixes comprising lactic acid producing bacteria that are dissolved when exposed to wet conditions. The film-shaped polymer matrixes protect bacterial cells from moisture thereby increasing bacterial survival during transport and storage. The film-shaped polymer matrix also results in a high transfer of bacterial cells to the skin of a subject.

There are several problems to solve to deliver microorganisms to the skin via an absorbent article. Incorporating a high dosage of microorganisms to the absorbent articles without significant loss of viability of the microorganisms, and minimizing viability loss of the microorganisms during production and storage of absorbent articles are critical. Transferring microorganisms in a desirable amount from the absorbent article to the skin is another problem to be solved.

There is therefore a need for an article containing microorganisms with the microorganisms being efficiently delivered to the skin such as the vaginal area under normal use conditions, such as those present during use of feminine hygiene products.

There is also a need for an article containing microorganisms which has a simpler construction compared to conventional absorbent articles.

There is also a need for an article containing microorganisms which can be applied in various types of absorbent articles or garments.

### SUMMARY OF THE INVENTION

The present invention fulfills the need described above by providing a patch comprising a dissolvable fibrous element comprising a filament-forming material and a microorganism, a liquid pervious first layer comprising a body facing surface and a garment facing surface opposite to the body facing surface, an adhesive disposed at least partially on the garment facing surface of the fist layer, and a removable release liner at least partially covering the adhesive.

The microorganism comprised in the patch according to the present invention exhibits less than a 3 log viability loss, or less than 2 log viability loss, or 1 log viability loss after being exposed to 25°C / 65% RH conditions for 4 weeks as measured according to the Viability Test Method defined herein.

In one embodiment, the microorganism exhibits less than a 3 log viability loss, or less than 2 log viability loss, or 1.5 log viability loss, or 1 log viability loss after being exposed to 25°C / 65% RH conditions for 8 weeks as measured according to the Viability Test Method defined herein.

In one embodiment, the patch contains at least 10³ CFU, or 10⁵ CFU, or 10⁷ CFU, or 10⁹ CFU, or 10¹¹ CFU per patch basis of the microorganism after being exposed to 25°C / 65% RH conditions for 4 weeks as measured according to the Viability Test Method defined herein.

In one embodiment, the patch transfers at least 10³ CFU, or 10⁴ CFU, or 10⁵ CFU, or 10⁶ CFU, or 10⁷ CFU, or 10⁸ CFU, or 10⁹ CFU of the microorganism as measured according to the In vitro Microorganism Transfer Test Method defined herein.

The present invention is also directed to a kit comprising at least one patch according to the present invention and at least one absorbent article.

These and other features, aspects, and advantages of the present invention will become evident to those skilled in the art from a reading of the present disclosure.

### BRIEF DESCRIPTION OF THE DRAWINGS

Fig. 1A is a top view of an exemplary patch according to the present invention.
Fig. 1B is a cross section view of the patch of Fig. 1A.
Fig. 1C shows an exemplary application of the patch according to the present invention.
Fig. 2 is a schematic representation of an example of a process for making a filament useful for the present invention.
Fig. 3 is a schematic representation of an example of a die suitable for use in the process in Fig. 2.
Fig. 4 is a front elevational view of a set-up for the Dissolution Test Method.
Fig. 5 is a partial top view of Fig. 4.
Fig. 6A is a plan view of a set-up for the In vitro Microorganism Transfer Test Method.
Fig. 6B is a cross-sectional representation of a portion of the apparatus in A-A direction cross section of the set-up in Fig. 6A.

### DETAILED DESCRIPTION OF THE INVENTION

All ranges are inclusive and combinable. The number of significant digits conveys neither limitations on the indicated amounts nor on the accuracy of the measurements.

The term "absorbent article(s)" as used herein, include disposable diapers, sanitary napkins, panty liners, incontinence pads, interlabial pads, animal-use excreta handling articles, animal-use diapers, tampons, sweat sheets, baby pants, toddler pants, overnight pants, and swim pants.

The term "additive(s)" as used herein means any material present in the dissolvable fibrous element of the present invention that is not a filament-forming material or a microorganism.

The term "benefit agents" as useful herein means agents having therapeutic benefit or their postulated mode of action. However, it is to be understood that the active and other ingredients useful herein can in some instances provide more than one therapeutic benefit or operate via more than one mode of action. Therefore, classifications herein are made for the sake of convenience and are not intended to limit an ingredient to the particularly stated application or applications listed.

The term "body facing surface" refers to the side of a patch or an absorbent article facing the body of the user when in use. The term "garment facing surface" refers to the opposite to surface of the patch or article.

The term "body fluid(s)," or "the fluid", used herein, includes, but is not limited to urine, menses, vaginal discharges, blood, sweat, and combinations of these substances.

"By weight on a dry fibrous element basis" means the weight of the fibrous element measured immediately after removing the fibrous element from a dessicator with a dessicant, for example a dessicator/dessicant commercially available from Desican Inc. under the tradename M-3003-66 which uses a molecular sieve dessicant, and in which the fibrous element has equilibrated in the dessicator for at least 24 hours prior to removing from the dessicator. The weight of the fibrous element is measured in a conditioned room at a temperature of 23°C ± 2.2°C and a relative humidity of 50% ± 10% immediately after removing the fibrous element from the dessicator/dessicant. In one example, "by weight on a dry fibrous element basis" means that the fibrous element may comprise less than 20%, or less than 15%, or less than 10%, or less than 7%, or less than 5%, or less than 3%, but greater than 0% by weight on a dry fibrous element basis of moisture, such as water, for example free water.

The term "dermatologically-acceptable," as used herein, means that the compositions or components thereof so described are suitable for use in contact with human skin without undue toxicity, incompatibility, instability, allergic response, and the like.

The term "fibrous element(s)" as used herein means an elongate particulate having a length greatly exceeding its average diameter, i.e. a length to average diameter ratio of at least about 10, including both filaments and fibers.

The term "filament(s)" as used herein means an elongate particulate having a length greater than or equal to 5.08 cm and/or greater than or equal to 7.62 cm and/or greater than or equal to 10.16 cm and/or greater than or equal to 15.24 cm.

The term "fiber(s)" as used herein means an elongate particulate having a length less than 5.08 cm and/or less than 3.81 cm and/or less than 2.54 cm.

The term "filament-forming composition(s)" as used herein means a composition that is suitable for making a filament of the present invention such as by meltblowing, dry spinning, and/or spunbonding. The filament-forming composition comprises a filament-forming material that exhibits properties that make it suitable for spinning into a filament. In one embodiment, the filament-forming material comprises a polymer. In addition to the filament-forming material, the filament-forming composition may comprise one or more additives such as a processing aid and a filler, and a material the absence of which from the filament would not result in the filament losing its filament structure, in other words, its absence does not result in the filament losing its solid form. In addition, the filament-forming composition may comprise one or more polar solvents, such as water, into which the filament-forming material and/or the microorganism and any additional additives, such as stabilizing agents and antioxidants, are dissolved and/or dispersed.

The term "filament-forming material(s)" as used herein means a material, such as a polymer or monomers capable of producing a polymer that exhibits properties suitable for making a filament. In one embodiment, the filament-forming material comprises one or more substituted polymers such as an anionic, cationic, zwitterionic, and/or nonionic polymer. In another embodiment, the polymer may comprise a hydroxyl polymer, such as a polyvinyl alcohol ("PVOH") and/or a polysaccharide, such as starch and/or a starch derivative, such as an ethoxylated starch and/or acid-thinned starch. In yet another embodiment, the filament-forming material is a polar solvent-soluble material.

The term "labile microorganism" as used herein means a microorganism that is likely to undergo change, for example a microorganism that is likely to lose all or a substantial part (at least a 1 log viability loss or greater as measured according to the Viability/Count Test Method described herein) when exposed to stresses, for example humidity, temperature, shear, aerobic conditions. A non-limiting example of a stress is exposing the microorganism to 25°C/60% RH conditions for 28 and/or 56 days. The *L. fermentum* in the Example below is an example of a labile microorganism as used herein.

The term "stabilizing agent(s)" as used herein means a material that improves the viability of microorganisms, for example by preventing and/or mitigating the dehydration of the microorganisms during and/or after the formation of the filament containing the microorganism.

The term "web(s)" as used herein means a collection of fibrous elements such as fibers and/or filaments of any nature or origin associated with one another.

### PATCH

The patch of the present invention comprises a dissolvable fibrous element comprising a filament-forming material and a microorganism, a liquid pervious first layer comprising a body facing surface and a garment facing surface opposite to the body facing surface, an attachment means disposed at least partially on the garment facing surface of the first layer, and optionally a removable release liner covering at least partially the attachment means when the attachment means comprises an adhesive. The microorganism in the patch according to the present invention exhibits less than a 3 log viability loss after the patch is exposed to 25°C and 65% relative humidity conditions for 4 weeks as measured according to the Viability Test Method. In one embodiment, the first layer can comprise the dissolvable fibrous element comprising a filament-forming material and a microorganism. The patch can be applied onto an absorbent article or underwear such as panties in the area where delivery of microorganisms to a targeted area of the skin is intended, such as the vaginal opening.

The patch of the present invention may not have a liquid impervious layer which blocks liquid flow except the removable release liner. When the patch of the present invention is placed on a body facing surface of the absorbent article in use, it is permeable to body fluid and does not interfere with the function of the absorbent article.

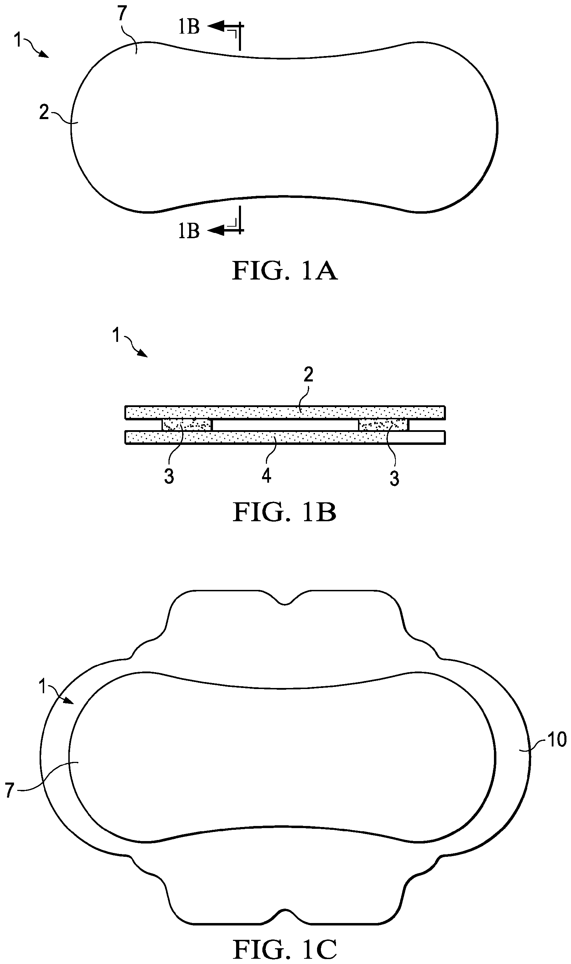

Figs. 1A and 1B show patch 1 of the present invention and a cross section thereof as indicated by section A-A of FIG. 1A, respectively, comprising a first layer 2, a body facing surface 7, adhesive 3 and a removable release liner 4. The patch may also be provided with additional features commonly found in sanitary napkins, including "wings" or "flaps" as is known in the art.

Fig. 1C shows one exemplary application of the patch 1 of the present invention onto an absorbent article 10. The patch of the present invention can be utilized by removing the removable releasing liner and placing the patch in an absorbent article or undergarment so that a garment facing surface of the main body portion of the patch is placed onto a body facing surface of the absorbent article or inside of the undergarment, and the patch is adhesive on the absorbent article or the undergarment using an attachment means disposed in the garment facing surface of the patch.

In some embodiments, the patch of the present invention may further comprise a liquid pervious second layer comprising a body facing surface disposed onto the body facing surface of the first layer. In one embodiment, the second layer can comprise the dissolvable fibrous element comprising a filament-forming material and a microorganism. In another embodiment, the fibrous element is disposed between the first layer and the second layer in a form of fibers.

In some embodiments, the patch of the present invention may further comprise a liquid pervious third layer disposed onto the body facing surface of the second layer. In one embodiment, the second layer can comprise the dissolvable fibrous element comprising a filament-forming material and a microorganism. In another embodiment, the fibrous element is disposed between the second layer and the third layer in a form of fibers

In some embodiments, the patch may comprise a plurality of two or more and/or three or more fibrous elements that are inter-entangled. In another embodiment, the patch may comprise water-soluble fibrous elements comprising one or more microorganisms. In another embodiment, the patch may comprise one or more dissolvable fibrous elements comprising one or more microorganisms, and one or more fibrous elements void of microorganisms. In another embodiment, the patch may comprise one or more water-soluble fibrous elements comprising one or more microorganisms, and one or more water-insoluble fibrous elements. In another embodiment, the patch may comprise two or more dissolvable fibrous elements wherein the fibrous elements release the microorganisms at different rates. In another embodiment, the patch may comprise, in addition to the dissolvable fibrous element comprising microorganisms according to the present invention, at least one of solid additives such as pulp fibers and particulates agent, and/or at least one benefit agent such as a prebiotic, an organic acid or acidic polymer, a skin care active, a stabilizing agent, an antioxidant, a plasticizer, a colorant and TSST-1 reducing material. In this case, one of the benefit agents can be incorporated either in the dissolvable fibrous element according to the present invention or in other additional fibrous elements. In some examples, one of the benefit agents can be incorporated either in the dissolvable fibrous element of the present invention or in other additional fibrous elements.

The dissolvable fibrous element comprising a filament-forming material and a microorganism may be present in a patch in the form of separate filaments and/or fibers relative to as an element of a web, by a method known in the art. The fibrous element in the form of separate filaments and/or fibers may be directly incorporated to the patch by disposing the fibrous element into a designated place of a patch using adhesives such as glue to keep the fibrous element in place. The separate fibrous element may be directly incorporated into a patch by disposing the fibrous element between two layers, and bonding the two layers together at several locations to trap the fibrous element between the layers. The fibrous element may be directly incorporated between two layers by means of electrostatic addition. Instead of adhesives, other materials that could hold the fibrous element in place without being an adhesive can be used.

The fibrous element may be directly incorporated to the patch by flocking, a technique by which fibers are fixed in a predetermined position on one layer forming the patch. US patent 6,497,688 discloses a method for flocking fibers on one or more of the internal surface of a patch and several references. Typically, all or a portion of a surface of a layer where fibers are disposed is coated with adhesive. The coated layer is then passed through a fiber metering station in which an electrostatic field is maintained around the layer, using for example electrodes situated above and below the layer. The fibers are applied to the adhesive on the layer in the presence of the electrostatic field, which orients the fibers perpendicular to the layer as they contact the adhesive. The layer is then heated, polymerizing the adhesive and anchoring the fibers. Unattached fibers may be vacuumed away.

Fibers can also be cut into particle like size, where a length of the fiber particle is not greater than 3 times the diameter of the fiber particle. In one embodiment, particles can be applied directly to the article by particle printing processes. Examples include gravure printing for super absorbent material printing and electrostatic printing. In another embodiment, fiber particles can be incorporated into an absorbent core by a process employed to add super absorbent materials into an absorbent core. In another embodiment, fiber particles can be "sprinkled" onto the article using adhesives to keep the particles in place. Besides adhesives, it is possible to use other materials that could act as a "hold in place" material without an adhesive. For example, when a lotion is added, fiber particles can be added on top of the lotion after the lotion is applied on a layer of a patch. Besides lotion, a viscous carrier such as PEG, PPG, silicone copolymers (i.e. DC-190), pluronics, lipid compounds (i.e. Akogel), and others can be used. In another embodiment, particles and other materials can be incorporated into other fluids to be applied by printing, slot coating, and spray on applications. Fluid examples include, PEGs, PPGs, silicone copolymers (dimethicones, i.e. OFX-0190 from Xiameter), lipids (i.e. petrolatum and Akogel from AKK), pluronics, lotions (petrolatum), and others. The selection of the carrier will depend on the application process and the selected active. As one example, OFX-0190 is a good candidate for application using a non-contact spray applicator. For example, the filament can be cut into small particles which are dispersed into the carrier at 10% solids or more (up to 60%). The mixture can be added to a tank that has an agitator to keep the mixture suspended. The mixture is then sprayed using an adhesive/glue applicator or a spray applicator onto a surface of a layer constituting a patch. The material can be applied to the area over the vagina' opening, or in stripes in the optimal zones to maximize transfer without impacting fluid handling. Other possible applications include slot coating and extrusion.

In one embodiment the fibrous element may be applied in a lotion for a patch to keep the fibrous element in place with or without adhesive. In one embodiment, the dissolvable fibrous element comprising a filament-forming material and a microorganism, regardless it is fibrous elements per se or an element of a layer, may be located below a layer directly facing a wearer's body which may reduce wearer's feeling a sticky sensation which dissolution of water soluble fibrous elements may cause. In another embodiment, the dissolvable fibrous element comprising a filament-forming material and a microorganism, regardless it is fibrous elements per se or an element of a layer, may be disposed on a body-facing surface of a topsheet.

In one aspect, the patch according to the present invention comprises a dissolvable fibrous element comprising a filament-forming material and a microorganism such that the microorganism exhibits less than a 3 log viability loss, or less than 2 log viability loss, or 1 log viability loss after being exposed to 25°C / 65% RH conditions for 4 weeks as measured according to the Viability Test Method defined herein.

In another aspect, the patch according to the present invention comprises a dissolvable fibrous element comprising a filament-forming material and a microorganism such that the microorganism exhibits less than a 3log viability loss, or less than 2 log viability loss, or 1.5 log viability loss, or 1 log viability loss after being exposed to 25°C / 65% RH conditions for 8 weeks as measured according to the Viability Test Method defined herein.

In another aspect, the patch according to the present invention comprises a region comprising a dissolvable fibrous element comprising a filament-forming material and a microorganism wherein the region contains at least 10³ CFU, or 10⁵ CFU, or 10⁷ CFU, or 10⁹ CFU, or 10¹¹ CFU of the microorganism on a basis of a gram of the patch after being exposed to 25°C / 65% RH conditions for 4 weeks as measured according to the Viability Test Method defined herein.

In another aspect, the patch according to the present invention comprises a region comprising a dissolvable fibrous element comprising a filament-forming material and a microorganism wherein the region contains at least 10³ CFU, or 10⁴ CFU, or 10⁴ CFU, or 10⁶ CFU of the microorganism on a basis of a gram of the patch after being exposed to 25°C / 65% RH conditions for 8 weeks as measured according to the Viability Test Method defined herein.

In another aspect, the patch according to the present invention transfers at least 10³ CFU, or 10⁴ CFU, or 10⁵ CFU, or 10⁶ CFU, or 10⁷ CFU, or 10⁸ CFU, or 10⁹ CFU of the microorganism per patch as measured according to the In vitro Microorganism Transfer Test Method defined herein.

The patch of the present invention may be produced industrially by any suitable means. The different layers may thus be assembled using standard means such as embossing, thermal bonding, or gluing or combination of both.

### Dissolvable Fibrous Element

The dissolvable fibrous element of the present invention comprises a filament-forming material and a microorganism. The total level of filament-forming materials and total level of microorganisms present in a filament-forming composition to produce the fibrous element may be any suitable amount so long as the fibrous element of the present invention is produced therefrom.

The dissolvable fibrous element of the present invention may be a filament and/or a fiber.

Filaments are typically considered continuous or substantially continuous in nature. The filaments of the present invention may be spun from filament-forming compositions via suitable spinning process operations, such as meltblowing, dry spinning, and/or spunbonding. The filaments of the present invention may be monocomponent and/or multi component. For example, the filaments may comprise bicomponent filaments. The bicomponent filaments may be in any form, such as side-by-side, core and sheath, islands-in-the-sea and the like.

Fibers are typically considered discontinuous in nature relative to filaments, which are considered continuous in nature. Non-limiting examples of fibers of the present invention include staple fibers produced by spinning a filament or filament tow of the present invention and then cutting the filament or filament tow into segments of less than 5.08 cm thus producing fibers. Fibers may be formed from a filament, such as when the filaments are cut to shorter lengths (such as less than 5.08 cm in length). Thus, in one embodiment, fibers in the present invention include fibers made from a filament of the present invention, such as a fiber comprising a filament-forming material and a microorganism. Therefore, references to filament and/or filaments of the present invention herein also include fibers made from such filament and/or filaments unless otherwise noted.

In one embodiment, the fibrous element may be single fibrous element rather than a yarn comprising a plurality of fibrous elements.

In another embodiment, the fibrous element may be hollow fibrous elements prior to and/or after release of one or more of microorganisms.

The fibrous element of the present invention may be hydrophilic or hydrophobic. The fibrous element may be surface treated and/or internally treated to change the inherent hydrophilic or hydrophobic property of the fibrous element.

In one embodiment, the total level of filament-forming materials present in the dissolvable fibrous element of the present invention is less than 90% and/or less than 80% and/or less than 70% and/or less than 60% by weight on a dry fibrous element basis and the total level of the one or more microorganisms present in the fibrous element is less than 50% and/or greater than 1% by weight on a dry fibrous element basis.

In one embodiment, the dissolvable fibrous element of the present invention may further comprise one or more additives such as a filler which is a material the absence of which would not result in the filament losing its filament structure, and/or at least one of benefit agents such as prebiotic, an organic acid or acidic polymer, a skin care active, a stabilizing agent, an antioxidant, a plasticizer, a colorant and a Toxic Shock Syndrome Toxin-1 (TSST-1) reducing material.

In another embodiment, the dissolvable fibrous element of the present invention comprises from about 20% and/or from about 30% and/or from about 40% to about 50% and/or to about 60% and/or to about 70% by weight on a dry fibrous element basis of a filament-forming material, such as polyvinyl alcohol polymer and/or a starch polymer, and at least 10³ CFU/g, or at least 10⁴ CFU/g, or at least 10⁵ CFU/g, or at least 10⁶ CFU/g, or at least 10⁷ CFU/g, or at least 10⁸ CFU/g on a dry fibrous element basis of microorganisms after being exposed to 25°C / 65% RH conditions for 4 weeks as measured according to the Viability Test Method defined herein.

In one embodiment, the dissolvable fibrous element exhibits an average diameter of less than 100 µm and/or less than 75 µm and/or less than 50 µm and/or less than 25 µm and/or less than 20 µm and/or less than 15 µm and/or less than 10 µm and/or greater than 1 µm and/or greater than 3 µm and/or greater than 5 µm and/or greater than 7 µm as measured according to the Diameter Test Method described herein. In another embodiment, the dissolvable fibrous element of the present invention exhibits an average diameter of greater than 1 µm as measured according to the Diameter Test Method described herein. The diameter of the dissolvable fibrous element of the present invention may be used to control the rate of release of one or more microorganisms present in the fibrous element and/or the rate of loss and/or altering of the fibrous element's physical structure.

In another embodiment, the dissolvable fibrous element of the present invention exhibits an average dissolution time of less than 60 minutes as measured according to the Dissolution Test Method described herein, is provided.

The dissolvable fibrous element in the present invention may comprise two or more different microorganisms. In one embodiment, the dissolvable fibrous element comprises two or more different microorganisms, wherein the two or more different microorganisms are compatible with one another. In another embodiment, the dissolvable fibrous element comprises two or more different microorganisms, wherein the two or more different microorganisms are incompatible for example in terms of food sources, etc.

In one embodiment, the dissolvable fibrous element may comprise microorganisms both within the fibrous element and microorganisms on an external surface of the fibrous element, such as a surface coating or partially embedded in the filament. The microorganism on the external surface of the fibrous element may be the same or different from the microorganism present in the fibrous element. If different, the microorganisms may be compatible or incompatible with one another.

In one embodiment, one or more microorganisms may be uniformly distributed or substantially uniformly distributed throughout the dissolvable fibrous element. In another embodiment, one or more microorganisms may be distributed as discrete regions within the fibrous element such that one portion of the fibrous element contains microorganisms and another portion of the fibrous element is void of microorganisms. In still another embodiment, at least a first microorganism is distributed uniformly or substantially uniformly throughout the fibrous element and a second microorganism different from the first microorganism is distributed as one or more discrete regions within the fibrous element. In still yet another embodiment, at least a first microorganism is distributed as one or more discrete regions within the fibrous element and a second microorganism different from the first microorganism is distributed as one or more discrete regions different from the first discrete regions within the fibrous element. In even another embodiment, the dissolvable fibrous element of the present invention may contain one or more microorganisms such that a cross-section of the fibrous element comprises at least two microorganisms. Still yet another embodiment of the dissolvable fibrous element of the present invention is a bicomponent filament wherein the core contains microorganisms and the sheath is void of microorganisms or the core is void of microorganisms and the sheath contains microorganisms or the core contains a first microorganism and the sheath contains a second microorganism different from the first microorganism or the core contains one or more microorganisms and the sheath contains one or more filament-forming materials. In another embodiment of a bicomponents filament, such as a side-by-side bicomponent filament, one side may contain a microorganism and the other side may be void of microorganisms or one side may contain a first microorganism and the other side may contain a second microorganism different from the first microorganism.

In one embodiment, the dissolvable fibrous element of the present invention is water-soluble.

In one embodiment, the dissolvable fibrous element of the present invention is such that one or more microorganisms may be present in the filament rather than on the filament, such as a coating on an exterior surface of the filament, such as in the form of a coating. A cross-section of the dissolvable fibrous element may comprise two or more microorganisms.

### Filament-forming Material

The dissolvable fibrous element of the present invention comprises one or more filament-forming materials. The filament-forming material may be present in the fibrous element at a total level of from about 10% to about 90%, or from about 20% to about 80%, or from about 30% to about 70%, or from about 40% to about 60% by weight on a dry fibrous element basis.

In one embodiment, the filament-forming material may comprise a polar solvent-soluble material, such as an alcohol-soluble material and/or a water-soluble material. Non-limiting examples of polar solvent-soluble materials include polar solvent-soluble polymers. The polar solvent-soluble polymers may be synthetic or natural in origin and may be chemically and/or physically modified. In one embodiment, the polar solvent-soluble polymers exhibit a weight average molecular weight of at least 10,000 g/mol and/or at least 20,000 g/mol and/or at least 40,000 g/mol and/or at least 80,000 g/mol and/or at least 100,000 g/mol and/or at least 1,000,000 g/mol and/or at least 3,000,000 g/mol and/or at least 10,000,000 g/mol and/or at least 20,000,000 g/mol and/or to about 40,000,000 g/mol and/or to about 30,000,000 g/mol.

In one embodiment, the water-soluble hydroxyl polymer is selected from the group consisting of polyvinyl alcohols, hydroxymethylcelluloses, hydroxyethylcelluloses, hydroxypropylmethylcelluloses and mixtures thereof. A non-limiting example of a suitable polyvinyl alcohol includes those commercially available from Sekisui Specialty Chemicals America, LLC (Dallas, TX) under the CELVOL® trade name. A non-limiting example of a suitable hydroxypropylmethylcellulose includes those commercially available from the Dow Chemical Company (Midland, MI) under the METHOCEL® trade name including combinations with above mentioned hydroxypropylmethylcelluloses.

In one embodiment, the polyvinyl alcohols herein can be grafted with other monomers to modify its properties. A wide range of monomers has been successfully grafted to polyvinyl alcohol. Non-limiting examples of such monomers include vinyl acetate, styrene, acrylamide, acrylic acid, 2-hydroxyethyl methacrylate, acrylonitrile, 1,3-butadiene, methyl methacrylate, methacrylic acid, maleic acid, itaconic acid, sodium vinylsulfonate, sodium allylsulfonate, sodium methylallyl sulfonate, sodium phenylallylether sulfonate, sodium phenylmethallylether sulfonate, 2-acrylamido-methyl propane sulfonic acid (AMPs), vinylidene chloride, vinyl chloride, vinyl amine and a variety of acrylate esters.

In yet another embodiment, the filament-forming material may be a film-forming material. In still yet another embodiment, the filament-forming material may be synthetic or of natural origin and it may be chemically, enzymatically, and/or physically modified.

In even another embodiment of the present invention, the filament-forming material may comprise a polymer selected from the group consisting of: polymers derived from acrylic monomers such as the ethylenically unsaturated carboxylic monomers and ethylenically unsaturated monomers, polyvinyl alcohol, polyacrylates, polymethacrylates, copolymers of acrylic acid and methyl acrylate, polyvinylpyrrolidones, polyalkylene oxides, starch and starch derivatives, pullulan, gelatin, hydroxypropylmethylcelluloses, methycelluloses, and carboxymethycelluloses.

In still another embodiment, the filament-forming material may comprises a polymer selected from the group consisting of: polyvinyl alcohol, polyvinyl alcohol derivatives, starch, starch derivatives, cellulose derivatives, hemicellulose, hemicellulose derivatives, proteins, sodium alginate, hydroxypropyl methylcellulose, chitosan, chitosan derivatives, polyethylene glycol, polyethylene oxide, polyacrylamide, tetramethylene ether glycol, polyvinyl pyrrolidone, hydroxymethyl cellulose, hydroxyethyl cellulose, and mixtures thereof.

In another embodiment, the filament-forming material comprises a polymer selected from the group consisting of: pullulan, hydroxypropylmethyl cellulose, hydroxyethyl cellulose, hydroxypropyl cellulose, polyvinyl pyrrolidone, carboxymethyl cellulose, sodium alginate, xanthan gum, tragacanth gum, guar gum, acacia gum, Arabic gum, polyacrylic acid, methylmethacrylate copolymer, carboxyvinyl polymer, dextrin, pectin, chitin, levan, elsinan, collagen, gelatin, zein, gluten, soy protein, casein, polyvinyl alcohol, starch, starch derivatives, hemicellulose, hemicellulose derivatives, proteins, chitosan, chitosan derivatives, polyethylene glycol, tetramethylene ether glycol, hydroxymethyl cellulose, and mixtures thereof.

In another embodiment, the filament-forming material is selected from the group consisting of: polyvinyl alcohol, polyvinyl alcohol derivatives, polyethylene oxide, starch, starch derivatives, cellulose, cellulose derivatives, carboxymethyl cellulose, hydroxypropylmethyl cellulose, hydroxyethyl cellulose, hydroxypropyl cellulose, polyvinyl pyrrolidone, sodium alginate, xanthan gum, tragacanth gum, guar gum, acacia gum, Arabic gum, polyacrylic acid, methylmethacrylate copolymer, carboxyvinyl polymer, chitosan, chitosan derivatives, polyethylene glycol, hemicellulose, hemicelluloses derivatives, polyacrylamide, and copolymers and mixtures thereof.

In one embodiment, the polar solvent-soluble polymers are selected from the group consisting of: alcohol-soluble polymers, water-soluble polymers and mixtures thereof Non-limiting examples of water-soluble polymers include water-soluble hydroxyl polymers, water-soluble thermoplastic polymers, water-soluble biodegradable polymers, water-soluble non- biodegradable polymers and mixtures thereof. In one embodiment, the water-soluble polymer comprises polyvinyl alcohol. In another embodiment, the water-soluble polymer comprises carboxymethylcellulose. In another embodiment, the water-soluble polymer comprises starch. In yet another embodiment, the water-soluble polymer comprises polyvinyl alcohol and starch.

### Microorganism

The dissolvable fibrous element of the present invention comprises one or more microorganisms, especially labile microorganisms. The microorganism may be selected from the group consisting of: prokaryotes, eukaryotes, viruses, bacteriophages, and mixtures thereof. Non-limiting examples of prokaryotes include bacteria and archaea. Non-limiting examples of eukaryotes include fungi.

### Bacteria

Bacteria suitable for use in the present invention include gram-positive cocci, gram-positive bacilli and gram-negative baccili. Non-limiting examples of bacteria for use in the dissolvable fibrous element of the present invention include microbes isolated from human and animal microbiota. In one embodiment, the bacteria is a probiotic.

Probiotics are bacteria that provides a beneficial health and/or welfare effect on its host, such as humans and/or animals. Non-limiting examples of probiotics for the present invention include Bifidobacteria species, Lactobacillus species, Lactococcus species, Pediococcus species, Leuconostoc species, Sporolactobacillus species, and Bacillus species and mixtures thereof.

Non-limiting examples of Bifidobacteria species include Bifidobacterium adolescentis, Bifidobacterium bifidum, Bifidobacterium animalis, Bifidobacterium thermophilum, Bifidobacterium breve, Bifidobacterium ion gum, Bifidobacterium infantis, Bifidobacterium lactis, Bifidobacterium longum, and Bifidobacterium pseudolongum. Specific strains of Bifidobacteria useful as probiotics include Bifidobacterium breve strain Yakult, Bifidobacterium breve R070, Bifidobacterium lactis Bb12, Bifidobacterium longum R023, Bifidobacterium bifidum R071, Bifidobacterium infantis 35624, Bifidobacterium infantis R033, Bifidobacterium longum BB536, Bifidobacterium animalis AHC7, and Bifidobacterium longum SBT-2928.

Non-limiting examples of Lactobacillus species for the present invention include Lactobacillus sporogenes, Lactobacillus jensenii, Lactobacillus vaginalis, Lactobacillus gallinarum, Lactobacillus coleohominis, and Lactobacillus iners, Lactobacillus bulgaricus, Lactobacillus cereale, Lactobacillus delbrukeii, Lactobacillus rhamnosus, Lactobacillus thermophilus, Lactobacillus paracasai sp. paracasai, Lactobacillus helveticus, Lactobacillus acidophilus, Lactobacillus brevis, Lactobacillus ulgaricus, Lactobacillus casei, Lactobacillus cellobiosus, Lactobacillus crispatus, Lactobacillus curvatus, Lactobacillus fermentum, Lactobacillus GG (Lactobacillus rhamnosus or Lactobacillus casei subspecies rhamnosus), Lactobacillus gasseri, Lactobacillus johnsonii, Lactobacillus plantarum, Lactobacillus reuteri, Lactobacillus salivarius, and mixtures thereof. Lactobacillus plantarum 299v strain originates from sour dough. Lactobacillus plantarum itself is of human origin. Other probiotic strains of Lactobacillus are Lactobacillus acidophilus BG2FO4, Lactobacillus acidophilus INT-9, Lactobacillus plantarum ST3 1, Lactobacillus reuteri, Lactobacillus johnsonii LA1, Lactobacillus acidophilus NCFB 1748, Lactobacillus casei Ski rota, Lactobacillus acidophilus NCFM, Lactobacillus acidophilus DDS-1, Lactobacillus delbrueckii subspecies delbrueckii, Lactobacillus delbrueckii subspecies bulgaricus type 2038, Lactobacillus acidophilus SBT-2062, Lactobacillus brevis, Lactobacillus salivarius UCC 118, Lactobacillus fermentum 297R1, Lactobacillus reuteri Grant L1, Lactobacillus crispatus 330L1, Lactobacillus and Lactobacillus paracasei subsp paracasei F 19. In one embodiment the microorganism comprises a species of Lactobacillus include L. caesi, L. acidophilus, L. plantarum, and L. rhamnosus.

Non-limiting examples of Lactococcus species for the present invention include Lactococcus lactis.

Non-limiting examples of Pediococcus species for the present invention include Pediococcus acidilactici, Pedioccocus pentosaceus, Pedioccocus urinae, Pediococcus cerevisiae, and mixtures thereof.

Non-limiting examples of Leuconostoc species for the present invention include Leuconostoc mesenteroides.

Non-limiting examples of Sporolactobacillus species for the present invention include Sporolactobacillus inulinus.

Non-limiting examples of Bacillus species for the present invention include Bacillus coagulans, Bacillus subtilis, Bacillus laterosporus, Bacillus laevolacticus, and mixtures thereof.

Other probiotic microbes that may be present in the dissolvable fibrous element of the present invention include Streptococcus species, Saccharomyces species, Enterococcusfaecium species and mixtures thereof.

Non-limiting examples of Streptococcus species for the present invention include Streptococcus lactis, Streptococcus cremoris, Streptococcus diacetylactis and Streptococcus thermophilus.

Non-limiting examples of Saccharomyces species for the present invention include Saccharomyces boulardii.

Non-limiting examples of Enterococcusfaecium species for the present invention include Enterococcusfaecium SF68.

In one embodiment, the probiotic is BifantisTM35624 (bifido bacterium, Chr. Hansen, Denmark) and/or Bifidobacterium infantis 35624. Non-limiting examples of other suitable probiotics include probiotics from strains of Bifidobacterium isolated from resected and washed human gastrointestinal tract. An example includes Bifidobacterium infantis strain designated UCC35624, described as being deposited at the National Collections of Industrial and Marine Bacteria Ltd (NCIMB) on Jan. 13, 1999, and accorded the accession number NCIMB 41003 and described in U.S. Pat. No. 7,195,906. Suitable examples of probiotics useful herein comprise strains of Bifidobacterium longum infantis (NCIMB 35624), Lactobacillus johnsonii (CNCM 1-1225), Bifidobacterium lactis (DSM20215), Lactobacillus paracasei (CNCM 1-2216), and mixtures thereof. Further non-limiting examples of probiotics useful herein are described in WO 03/010297 A1, WO 03/010298 A1, WO 03/010299 A1 (all published Feb. 6, 2003 and assigned to Alimentary Health Ltd) and US Patent Application Publication No. US2012/0276143.

In one embodiment, the probiotic comprises Bifidobacterium strain AH1714 and/or Bifidobacterium longum strain UCC35624. A deposit of Bifidobacterium longum strain AH1714 was made at the National Collections of Industrial and Marine Bacteria Limited (NCIMB) Ferguson Building, Craibstone Estate, Bucksbum, Aberdeen, AB21 9YA, Scotland, UK on Nov. 5, 2009 and accorded the accession number NCIMB 41676. A deposit of Bifidobacterium longum strain UCC35624 was made at the National Collections of Industrial and Marine Bacteria Limited (NCIMB) Ferguson Building, Craibstone Estate, Bucksburn, Aberdeen, AB21 9YA, Scotland, UK on Jan. 13, 1999 and accorded the accession number NCIMB 41003. The Bifidobacterium longum strain may be a genetically modified mutant or it may be a naturally occurring variant thereof. In one embodiment, the Bifidobacterium longum strain is in the form of viable cells. In another embodiment, the Bifidobacterium longum strain is in the form of non-viable cells

In another embodiment, the probiotic comprises Bifidobacterium strain AH121A. A deposit of Bifidobacterium longum strain AH121A was made at the National Collections of Industrial and Marine Bacteria Limited (NCIMB) Ferguson Building, Craibstone Estate, Bucksburn, Aberdeen, AB21 9YA, Scotland, UK on Nov. 5, 2009 and accorded the accession number NCIMB 41675.

In another embodiment, the probiotic comprises Bifidobacterium strain AH121A. A deposit of Bifidobacterium longum strain AH121A was made at the National Collections of Industrial and Marine Bacteria Limited (NCIMB) Ferguson Building, Craibstone Estate, Bucksburn, Aberdeen, AB21 9YA, Scotland, UK on Nov. 5, 2009 and accorded the accession number NCIMB 41675.

Such probiotics, in one embodiment, can be present in the dissolvable fibrous element of the present invention at from about 0.025% to about 10% and/or from about 0.025% to about 5% and/or from about 0.025% to about 3% and/or from about 0.025% to about 1%, by weight on a dry fibrous element basis.

### Fungi

Non-limiting examples of suitable fungi for the present invention include Penicillum, Saccharomyces cerevisiae, and mixtures thereof.

### Viruses

Suitable viruses for the present invention include all 7 groups of viruses. These include Group I : double-stranded DNA viruses; Group II : single-stranded DNA viruses; Group III : double-stranded RNA viruses; Group IV : positive-sense single-stranded RNA viruses; Group V : negative-sense single-stranded RNA viruses; Group VI : reverse transcribing RNA viruses and Group VII: reverse transcribing DNA viruses. Non-limiting examples include vaccines for human and animals.

### Bacteriophages

Suitable bacteriophages for the present invention include Salmonella phases, E.coli phases, Pseudomonas phases, Bacillus phages, Listeria phases, Burkholderia phages, Staphylococcus aureus phases, and Streptococcus mutans phages.

### First Layer

The patch of the present invention comprises a liquid pervious first layer comprising a body facing surface and a garment facing surface opposite to the body facing surface.

The term "liquid pervious" as used herein refers to components that allow liquids to pass therethrough without significantly retarding or obstructing the transmission of such liquids therethrough. The first layer may be a polymeric film layer, nonwoven layer, or a composite or a laminate comprising a nonwoven layer or a polymeric film. When the first layer comprises the dissolvable fibrous element of the present invention, it preferably comprises a single-layered or multi-layered nonwoven web, or a laminate having a nonwoven layer comprising the dissolvable fibrous element of the present invention.

The first layer may comprise a nonwoven web comprising the dissolvable fibrous element of the present invention. The web comprising the fibrous element may be an arrangement comprising a plurality of two or more and/or three or more fibrous elements that are inter-entangled. The web may comprise a dissolvable fibrous element comprising a microorganism and a fibrous element void of microorganisms. The web may comprise a water soluble fibrous element comprising a microorganism and a water insoluble fibrous element. In this case, the ratio of water soluble fibrous elements to water insoluble fibrous elements may be controlled and determined to reduce the stickiness of the first layer resulting from dissolution of the water soluble fibrous element. The web may comprise, in addition to the dissolvable fibrous element of the present invention, at least one of solid additives such as pulp fibers and particulates agent, a prebiotic, an organic acid or acidic polymer, a skin care active, a stabilizing agent, an antioxidant, a plasticizer, a colorant and TSST-1 reducing material.

In this case, one of the benefit agents can be incorporated either in the dissolvable fibrous element according to the present invention or in other additional fibrous elements.

In one embodiment, the first layer when it comprises a fibrous element illustrated herein may exhibit an average Dissolution Time of less than 12 hours, or less than 8 hours, or less than 6 hours, or less than 2 hours, or, less than 1 hour, or less than 30 minutes, or less than 10 minutes, or less than 5 minutes, or less than 1 minute, or less than 30 seconds, or may be instantaneous as measured according to the Dissolution Test Method described herein. The nonwoven first layer can be designed to have an appropriate Dissolution Time depending on an application of a patch having the nonwoven first layer.

### Attachment Means

The present invention comprises an attachment means to affix the patch of the present invention to an absorbent article or the wearer's undergarment. The attachment means is applied in the garment facing surface of the patch, and is intended to hold in place the patch on a body facing side of an absorbent article or undergarment. Attachment means comprising an adhesive has been found to work well for these purposes. When the patch of the present has flaps or wings, the attachment means can be also applied to the garment facing surface of the flaps and wings as well as a patch body so as to contact and adhere to a body facing side of an absorbent article or undergarment.

### Adhesive

The present invention comprises an adhesive as an attachment means. The adhesives can comprise any adhesive or glue used in the art for such purposes. The adhesives typically are pressure sensitive and remain tacky well below their application temperature

The adhesive may be applied to the garment-facing surface of the patch using any one of methods well known in the art for this purpose such as slot coating, spraying and roll printing. One method of applying the adhesive to the garment facing surface of the patch is the direct coating; another method is printing the adhesive onto a removable release liner, which is then pressed onto the garment facing surface of the patch. Thereby the adhesive is transferred from the removable release liner to the garment facing surface of the patch. In an embodiment, the attachment means comprises a plurality of spaced apart longitudinally-oriented strips of adhesive.

It may be desirable that the adhesive has a surface coverage on the garment facing surface of the patch from about 5 to about 99%, or from about 10 to about 95%, or from about 10 to about 60% or from about 15 to about 50%.

The adhesive suitable for the present invention is preferably liquid pervious.

The adhesive attachment means is covered by a removable release liner further explained below.

### Removable Release Liner

A removable release liner is a disposable element which serves to protect the attachment means when it comprises an adhesive from contamination and from adhering to another surface prior to application. When the attachment means comprises adhesive, typically, the removable release liner is formed from a material impermeable to adhesive, and is easily stripped from the adhesive that are applied on an outmost surface, a garment facing surface. A removable release liner herein is understood to include a protective cover. A protective cover can be provided as a single piece or in a multitude of pieces, e.g. to cover the individual adhesive areas.

A removable release liner may comprise silicone coated release paper, a plastic film or any other easily removable cover.

A removable release liner also can perform other functions such as provision of individualised packaging for the patch or provide a disposal function. Any commercially available release paper or film may be used. Suitable examples include BL 30 MG-A SILOX EI/O, BL 30MG-A SILOX 4 P/O available from Akrosil Corporation, and M&W films available from Gronau in Germany, under the code X-5432.

### Benefit Agent

The patch according to the present invention may further comprise at least one benefit agent such as a prebiotic, an organic acid or acidic polymer, a skin care active, a stabilizing agent, an antioxidant, a plasticizer, a colorant and TSST-1 reducing material. It should be noted, however, that many benefit agents may provide more than one benefit. Therefore, classifications herein are made for the sake of convenience and are not intended to limit the additive to that particular application or applications listed.

In one embodiment, the patch of the present invention includes at least one of benefit agents. In another embodiment, the patch of the present invention comprising one layer having at least one benefit agent, and another layer having a different benefit layer.

### Prebiotic

The patch according to the present invention may further comprise a prebiotic. Suitable prebiotics can include one or more of a carbohydrate, carbohydrate monomer, carbohydrate oligomer, or carbohydrate polymer.

Non-limiting examples of suitable prebiotics include inulin, lactose, lactulose, raffinose, stachyose, fructooligossacharides, glucooligosaccharides, lactoferrin, mannan oligosaccharides, glucan oligosaccharides, isomaltooligosaccharides, lactosucrose, polydextrose, soybean oligosaccharides, xylooligosaccharides, paratinose oligosaccharides, transgalactosylated oligosaccharides, transgalactosylate disaccharides, gentiooligosaccharides, pecticoligosaccharides palatinose polycondensates, difructose anhydride III, sorbitol, maltitol, lactitol, polyols, polydextrose, reduced paratinose, cellulose, β-glucose, β-galactose, β-fructose, verbascose, galactinol, β-glucan, guar gum, pectin, sodium alginate, and lambda carrageenan and mixtures thereof.

Other non-limiting examples of suitable prebiotics include human milk oligosaccharides as disclosed in US2013281948 A1, for example lactose, 2'-fucosyllactose, 3'-fucosyllactose, difucosyllactose, lacto-N-tetraose (type 1), lacto-N-neo-tetraose (type 2), lacto-N-fucopentaoses I, II, III, IV and V, lacto-N-fucohexaose I, lacto-N-hexaose, lacto-N-neohexaose, fucosyllacto-Nhexaose I and IV, fucosyllacto-N-neohexaose, lacto-N-difuco-hexaoses I and II, lacto-Noctaoses, sialya2-3lactose, sialya2-6lactose, sialyl-lacto-N-tetraose a, b and c, and disialyl-lacto-N-tetraose, and mixtures thereof.

Still other non-limiting examples of suitable prebiotics include fucose-a(1®2)galalactose b as a disaccharide unit, 2'-fucosyllactose, 3'-fucosyllactose, lacto-N-difuco-tetraose, lacto-N-difuco-hexose I, lacto- N-difuco-hexose II, lacto-N-fucopentaose I, lacto-N-fucopentaose II, lacto-N-fucopentaose III, lacto-N-fucopentaose V, and mixtures thereof.

In one embodiment, the prebiotic may comprise carob bean, citrus pectin, rice bran, locust bean, fructooligosaccharide, oligofructose, galactooligosaccharide, citrus pulp, annanoligosaccharides, arabinogalactan, lactosucrose, glucomannan, polydextrose, apple pomace, tomato pomace, carrot pomace, cassia gum, gum karaya, gum talha, gum arabic, and combinations thereof. Such prebiotics, in one embodiment, may be present in the dissolvable fibrous element according to the present invention at from about 0.01% to about 30% and/or from about 0.1% to about 20% and/or from about 1% to about 15% by weight on a dry fibrous element basis.

### Organic acids and acidic polymers

The patch of the present invention may further comprise at least one organic acid or acidic polymer. When present in the dissolvable fibrous element according to the present invention, the organic acid and/or acidic polymer may be present at a level of from about 0.01% to about 30% and/or from about 0.1% to about 20% and/or from about 1% to about 15% by weight on a dry fibrous element basis.

Non-limiting examples of suitable organic acids include acetic acid, propionic acid, lactic acid, ascorbic acid, phenylalanine, citric acid, butyric acid, valeric acid, capronic acid, succinic acid, benzoic acid, alginic acid, sorbic acid, stearic acid, oleic acid, edetic acid, gluconodeltalactone, fumaric acid, malic acid, succinic acid, gluconic acid, ascorbic acid, tartaric acid, glycolic acid, and salts thereof. Additionally acidic polymers with ionizable functional groups including a carboxylic acid may also be used. An example of the acidic polymer is a polyacrylic polymer. The acidic polymers preferably used in this invention include Carbopols, available commercially from Lubrizol, Cleveland, Ohio.

### Skin care active

The patch of the present invention may further comprise at least one skin active. Non-limiting examples of suitable skin care actives include skin protectant actives (FDA: Skin Protectant Drug Products for Over-the-Counter Human Use; Final Monograph) such as allantoin, aluminum hydroxide gel, calamine, cocoa butter, colloidal oatmeal, dimethicone, cod liver oil (in combination), glycerine, hard, fat, kaolin, petrolatum, lanolin, mineral oil, shark liver oil, white petrolatum, sodium bicarbonate, topical starch, zinc acetate, zinc carbonate, zinc oxide, and the like.

Another non-examples of suitable skin care actives include actives disclosed in WO 2013/122932 as ingredients useful for regulating and/or improving a condition of mammalian skin such as vitamins; peptides and peptide derivatives; sugar amines; phytosterols; salicylic acids; hexamidines compounds; dialkanoyl hydroxyproline compounds, flavonoids, retinoid compounds, botanicals, N-acyl amino acid compounds; conditioning agents. The actives include salts thereof, derivatives thereof and combinations thereof.

Exemplary vitamins suitable for use herein may include one or more water-soluble vitamins. Examples of water-soluble vitamins including, but are not limited to, water-soluble versions of vitamin B, vitamin B derivatives, vitamin C, vitamin C derivatives, vitamin K, vitamin K derivatives, vitamin D, vitamin D derivatives, vitamin E, vitamin E derivatives, provitamins thereof, such as panthenol and mixtures thereof. Exemplary vitamins suitable for use herein may include a vitamin B3 compound and its derivatives such as niacinamide, nicotinic acid esters, including non-vasodilating esters of nicotinic acid (e.g., tocopheryl nicotinate, myristyl nicotinate).

Peptides may contain ten or fewer amino acids and their derivatives, isomers, and complexes with other species such as metal ions (e.g., copper, zinc, manganese, magnesium, and the like). Peptides suitable for use herein may include di-, tri-, tetra-, penta-, and hexa-peptides and derivatives thereof. Also useful herein as peptides are naturally occurring and commercially available compositions that contain peptides. Some examples of peptides include the dipeptide carnosine (beta-ala-his), the tripeptide gly-his-lys, the pentapeptide lys-thr-thr-lys-ser, lipophilic derivatives of peptides, and metal complexes of the above, e.g., copper complex of the tripeptide his-gly-gly (also known as Iamin). A commercially available tripeptide derivative-containing composition is Biopeptide CL®, which contains 100ppm of palmitoyl-gly-his-lys, and is commercially available from Sederma. A preferred commercially available pentapeptide derivative-containing composition is Matrixyl®, which contains 100ppm of palmnitoyl-lys-thr-thr-lys-ser and is commercially available from Sederma.

Exemplary sugar amines suitable for use herein are described in WO 02/076423 and U.S. Pat. No. 6,159,485. A particularly suitable example of a sugar amine is glucosamine and its salts, which may be found in certain shellfish or derived from fungal sources. Other examples of sugar amines include N-acetyl glucosamine, mannosamine, N-acetyl mannosamine, galactosamine, N-acetyl galactosamine, their isomers (e.g., stereoisomers), and their salts (e.g., HCl salt).

Exemplary hexamidines suitable for use herein may include hexamidine derivatives such as any isomers and tautomers of hexamidine compounds including, but not limited to, organic acids and mineral acids, for example sulfonic acid, carboxylic acid, etc. The hexamidine compounds include hexamidine diisethionate.

Flavonoids suitable for use herein broadly disclosed in U.S. Pat. Nos. 5,686,082 and 5,686,367. Examples of some flavonoids are one or more flavones, one or more isoflavones, one or more coumarins, one or more chromones, one or more dicoumarols, one or more chromanones, one or more chromanols, isomers (e.g., cis/trans isomers) thereof, and mixtures thereof. Some examples include flavones and isoflavones, such as daidzein (7,4'-dihydroxy isoflavone), genistein (5,7,4'-trihydroxy isoflavone), equol (7,4'-dihydroxy isoflavan), 5,7-dihydroxy-4'-methoxy isoflavone, soy isoflavones (a mixture extracted from soy), and mixtures thereof.

"Retinoid" as used herein means natural and synthetic analogs of Vitamin A, or retinol-like compounds which possess the biological activity of Vitamin A in the skin, as well as the geometric isomers and stereoisomers of these compounds. The retinoid may be selected from retinol, retinol esters (e.g., C2-C22 alkyl esters of retinol, including retinyl palmitate, retinyl acetate, retinyl propionate), retinal, and/or retinoic acid (including all-trans retinoic acid and/or 13-cis-retinoic acid), or mixtures thereof.

For N-acyl amino acid compounds suitable for use herein, the amino acid can be one of any of the amino acids known in the art. A list of possible side chains of amino acids known in the art are described in Stryer, Biochemistry, 1981, published by W.H. Freeman and Company. R1can be C1 to C30, saturated or unsaturated, straight or branched, substituted or unsubstituted alkyls; substituted or unsubstituted aromatic groups; or mixtures thereof. The N-acyl amino acid compound may be selected from the group consisting of N-acyl phenylalanine, N-acyl Tyrosine, their isomers, their salts, and derivatives thereof. The amino acid can be the D or L isomer or a mixture thereof. One embodiment of an amino acid derivative is N-undecylenoyl-L-phenylalanine, which belongs to the class of N-acyl phenylalanine amino acid derivatives. This exemplary amino acid derivative includes an acyl group which is a Cn mono-unsaturated fatty acid moiety and the L-isomer of phenylalanine. One embodiment of N-undecylenoyl-L-phenylalanine is commercially available under the tradename Sepiwhite® from SEPPIC.

Some non-limiting examples of conditioning agents such as a humectant, a moisturizer, or a skin conditioner include, but are not limited to, guanidine; urea; glycolic acid and glycolate salts (e.g. ammonium and quaternary alkyl ammonium); aloe vera in any of its variety of forms (e.g., aloe vera gel); polyhydroxy alcohols such as sorbitol, mannitol, xylitol, erythritol, glycerol, hexanetriol, butanetriol, propylene glycol, butylene glycol, hexylene glycol and the like; polyethylene glycols; sugars (e.g., melibiose) and starches; sugar and starch derivatives (e.g., alkoxylated glucose, fucose); hyaluronic acid; lactamide monoethanolamine; acetamide monoethanolamine; panthenol; allantoin; and mixtures thereof. Also useful herein are the propoxylated glycerols described in U.S. Pat. No. 4,976,953. Also useful are various C1-C30 monoesters and polyesters of sugars and related materials. These esters are derived from a sugar or polyol moiety and one or more carboxylic acid moieties.

When present in the dissolvable fibrous element according to the present invention, the skin care active may be present at a level of from about 0.01% to about 30% and/or from about 0.1% to about 20% and/or from about 1% to about 15% by weight on a dry fibrous element basis.

### Stabilizing Agent

The patch of the present invention may further comprise a stabilizing agent. When present in the dissolvable fibrous element according to the present invention, the stabilizing agent may be present at a level of from about 0.01% to about 30% and/or from about 0.1% to about 20% and/or from about 1% to about 15% by weight on a dry fibrous element basis. The stabilizing agent may comprise a carbohydrate and/or a protein.

The carbohydrate may be present in the dissolvable fibrous element at a level of from about 0% to about 50% and/or from about 10% to about 40% by weight on a dry fibrous element basis. The carbohydrate may be selected from the group consisting of: monosaccharides, disaccharides, oligosaccharides, polysaccharides, and mixtures thereof. Non-limiting examples of suitable carbohydrates include sucrose, trehalose, glycerol, glucose, mannitol, sorbitol, adonitol, betaine (N,N,N-trimethylglycine), lactose, fructo-oligosaccharides (FOS), polyfructoses, for example, inulin, pectin, 6-glucans, resistant starches, for example high amylose starch, dextrans, acacia gum, guar and locust bean gum, agar, carrageenans, xanthan and maltodextrins, and mixtures thereof.

When present in the dissolvable fibrous element according to the present invention, the protein may be present at a level of from about 0.01% to about 30% and/or from about 1% to about 20% by weight on a dry fibrous element basis. The protein may be selected from the group consisting of albumen, arginine/lysine polypeptide, collagen and hydrolyzed collagen, gelatin and hydrolyzed gelatin, glycoproteins, milk protein, casein, whey protein, soy protein, barley protein, serum albumin, meat, fish, seafood, poultry, egg proteins, silk, soybean, corn, peanut, cottonseed, sunflower, pea, wheat protein, wheat germ protein, gluten-protein, zein and any isolate or hydrolyzed of any vegetable protein, such as soy protein isolate and/or hydrosylate, barley protein isolate and/or hydrosylate, and mixtures thereof.

### Antioxidant

The patch of the present invention may further comprise an antioxidant. When present in the dissolvable fibrous element according to the present invention, the antioxidant may be present at a level of from about 0.01% to about 30% and/or from about 0.1% to about 20% and/or from about 1% to about 15% by weight on a dry fibrous element basis. Non-limiting examples of antioxidants for the present invention include the following.

Rice bran derivatives have been shown to have more than a hundred (100) potent antioxidants including vitamin E and its isomers (tocopherols (T) and tocotrienols (T3), collectively referred to as tocols. A tocol-rich substance is a mixture containing one or more compounds selected from tocopherols (T), tocotrienols, and tocotrienollike (T3 -like) compounds. Stabilized rice bran is the highest natural source of vitamin E.

Additional antioxidants in stabilized rice bran derivatives include, but are not limited to, y-oryzanol, (3-carotene, several known flavanoids, phytosterols, lipoic acid, ferulic acid and inositol hexaphospate (i.e., "IP6"). Some of these compounds are present in stabilized rice bran derivatives at concentrations which are much higher than in any of the known natural sources of the compounds. Ferulic acid, for example, is a phytochemical found in seeds of plants such as in brown rice, whole wheat and oats, as well as in coffee, apple, artichoke, peanut, orange and pineapple. Ferulic acid protects our cells form ultraviolet rays and neutralizes reactive oxygen species in the body, thereby preventing the reactive oxygen species from causing damage to our DNA. Being an antioxidant, it also reduces the level of cholesterol and triglyceride in the body and thus lowers the risk of heart diseases. IP6 is a phosphorylated form of inositol commonly found in fiber-rich plant foods. IP6 is hydrolyzed by phytase enzymes in the digestive tract to yield inositol. IP6 supports a cell's natural defense against damaging hydroxyl free radicals by chelating with reactive iron. In combination with probiotics, antioxidants provide exceptional additional defense and increase the immune system's ability to resist invasive pathogens associated with gastrointestinal disorders.

In one embodiment, the antioxidants present in the filaments may be selected from the group consisting of: carotenoids, such as lycopene, beta-carotene, lutein, xanthophylls, vitamin A, tocopherols, vitamin C, and mixtures thereof.

In another embodiment, the antioxidants present in the filaments may be selected from the group consisting of propyl gallate, butylated hydroxytoluene (BHT), butylated hydroxyanisole (BHA), Vitamin C, Vitamin A, Vitamin E, beta-carotene, and mixtures thereof.

### TSST-1 reducing material

The patch of the present invention may further comprise a TSST-1 reducing material. Non-limiting examples of TSST-1 reducing material can include, but are not limited to L-ascorbic acid, monoesters and diesters of polyhydric aliphatic alcohols such as glycerol monolaurate, and calcium salts such as calcium lactate, calcium citrate malate, and calcium compounds such as calcium sugar phosphates disclosed in WO 2010/129444. Still further examples have introduced to tampons non-ionic surfactants, such as alkyl ethers, alkyl amines, and alkyl amides as detoxifying compounds. When present in the dissolvable fibrous element according to the present invention, the TSST-1 reducing material may be present at a level of from about 0.01% to about 30% and/or from about 0.1% to about 20% and/or from about 1% to about 15% by weight on a dry fibrous element basis.

### KIT

The present invention is also directed to a kit comprising at least one patch according to the present invention and at least one absorbent article. In one embodiment, the patch is in the same size as or in a smaller size than the absorbent article.

The kit of the present invention may further comprise an outer package housing at least one patch of the present invention and at least one absorbent article as described herein. The patch and/or the absorbent article may be disposed in an inner package. The inner package includes a wrapper, pouch, box or bag in which one or more patches are placed. The inner package can be placed into the outer package.

The kit may contain a plurality of patches of the present invention together with one or a plurality of absorbent articles . The patch can be in the same size, or at least some of them can be in different sizes. At least some of the absorbent articles are for different types such as sanitary pads, tampons, pantyliners, adult incontinent products, and baby diaper. For example, one or more of the absorbent articles may be for nighttime use (sleep-use), and one or more for daytime use, with respect to features such as absorbent capacity.

In another embodiment, the kit comprises a patch according to the present invention and at least two different types of absorbent articles. The patch is in the same size as one of the absorbent articles, or in a smaller size than any of the absorbent articles.

The kit of the present invention may further comprise a set of instructions. In an embodiment, the set of instructions comprises instructions that direct a wearer or caregiver to use a regimen comprising applying a patch to a body facing surface of a absorbant article or a garment, and wearing the article or the garment to contact to the skin.

In certain embodiments, it may be desirable to use the patch of the present invention at one or more of the foregoing frequencies for at least two consecutive or nonconsecutive application periods.

### Absorbent Article

An absorbent article may comprise a topsheet; and a backsheet joined to the topsheet and optionally an absorbent core disposed between the topsheet and the backsheet.

### Topsheet

With the nonwoven according to the present invention, the first fiber layer is preferably, disposed on a side in contact with the skin.

### Backsheet

Any conventional backsheet materials commonly used for patchs may be used as backsheet. In some embodiments, the backsheet may be impervious to malodorous gases generated by absorbed bodily discharges, so that the malodors do not escape. The backsheet may or may not be breathable.

### Absorbent core

It may be desirable that the article further comprises an absorbent core disposed between the topsheet and the backsheet. As used herein, the term "absorbent core" refers to a material or combination of materials suitable for absorbing, distributing, and storing fluids such as urine, blood, menses, and other body exudates. Any conventional materials for absorbent core suitable for absorbent articles may be used as absorbent core.

### METHOD FOR PRODUCTION

### Filaments

The filaments comprising a filament-forming material and a microorganism can be produced by spinning a filament-forming composition comprising one or more filament-forming materials and one or more microorganisms.

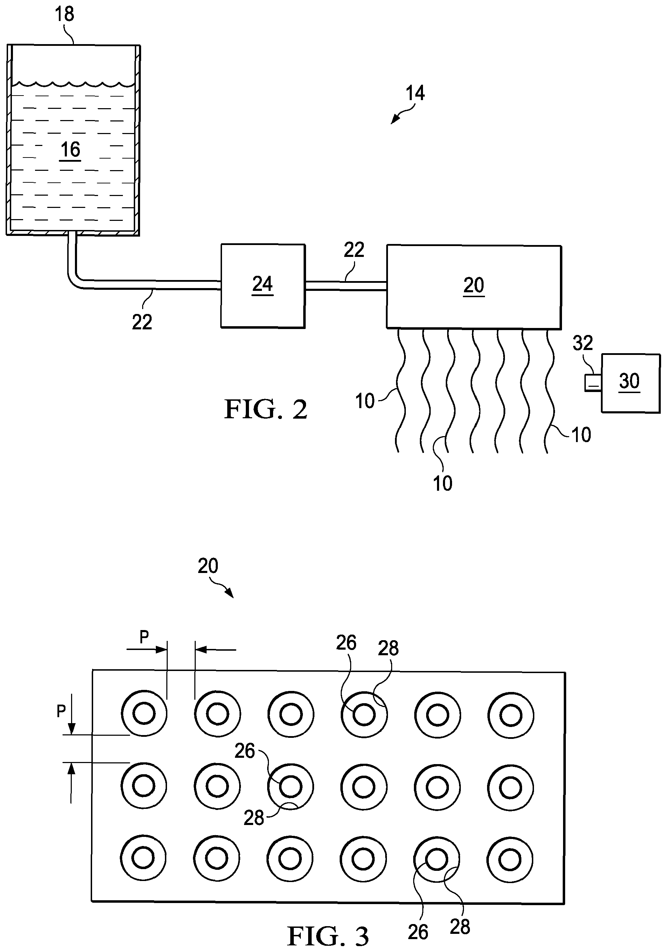

In one embodiment, as shown in Fig. 2, a method 14 for making a filament 10 of the present invention comprises the steps of:
a. providing a filament-forming composition 16, for example a filament-forming liquid composition suitable for making filaments, comprising one or more filament-forming materials, one or more microorganisms, and one or more stabilizing agents, from a source 18, such as a tank, for example a pressurized tank suitable for batch operations; and
b. spinning the filament-forming composition 16 from a die 20, such as a meltblow die, to produce one or more filaments 10 of the present invention.

The filament-forming composition 16 may be in fluid communication with the die 20 via suitable piping 22 as shown with the arrows. A pump 24 (for example a Zenith®, type PEP II pump having a capacity of 5.0 cubic centimeters per revolution (cc/rev), manufactured by Parker Hannifin Corporation, Zenith Pumps division, of Sanford, N.C., USA) may be used to pump the filament-forming composition 16 to the die 20. The filament-forming composition's flow to the die 20 may be controlled by adjusting the flow rate of the pump 24.

The die 20 as shown in Fig. 3 may comprise two or more rows of circular extrusion nozzles 26 spaced from one another at a pitch P of about 1.524 millimeters (about 0.060 inches). The nozzles 26 may have individual inner diameters of about 0.305 millimeters (about 0.012 inches) and individual outside diameters of about 0.813 millimeters (about 0.032 inches). Each individual nozzle 26 may be encircled by an annular and divergently flared orifice 28 to supply attenuation air formed by mixing steam and heated compressed air to each individual nozzle 26. The filament-forming composition 16 that is extruded through the nozzles 26 is surrounded and attenuated by generally cylindrical, attenuation air streams supplied through the orifices 28 encircling the nozzles 26 to produce the filaments 10. The filaments 10 may be dried by a drying air stream having a temperature of from about 50° C to about 315°C by an electrical resistance heater 30 supplied through drying nozzles 32 and discharged at an angle of about 90° relative to the general orientation of the filaments 10 being spun.

During spinning of the filament-forming composition, the filament-forming composition and microorganisms contained therein are subjected to steam and attenuation air and drying air at temperatures of up to 450°C without negatively impacting the viability of the microorganisms, for example with less than 3 log loss in viability, or less than a 2 log loss in viability of the microorganisms. The filaments 10 may be collected on a collection device, such as a belt or fabric, in one embodiment a belt or fabric capable of imparting a pattern, for example a non-random repeating pattern to a web formed as a result of collecting the filaments on the belt or fabric.

In one embodiment, the step of spinning may comprise contacting the filament with attenuation air to attenuate the filament.

The method may further comprise the step of collecting a plurality of filaments on a collection device, for example a spool or a belt or fabric, such as a patterned belt. Filaments may be collected and stored in desiccated flip top vials (commercially available from Desican Inc) and refrigerated until use.

The filaments of the present invention may exhibit an average diameter of greater than 1µm and/or greater than 3µm and/or greater than 5µm and/or less than 100µm and/or less than 70 µm.

In one embodiment, the method of the present invention is a non-electrospinning method.

### Fibers

Filaments can be cut into a predetermined length to obtain fibers by methods known in the art.

### Nonwoven Web

A nonwoven web comprising filaments comprising a filament-forming material and a microorganism can be formed by a process conventional in the art such as meltblowing, spunlaid, solvent spinning, electrospinning, airlaying, dry laying, wetlaying with staple fibers, and carding. As used herein, "spunlaid" refers to fibers made by spunbond. The basis weight of nonwoven web is usually expressed in grams per square meter (gsm). The nonwoven web obtained is used or can be cut into a predetermined size to be used as a first layer.

### Patches

Patches in the present invention may comprise the usual layers or components normally found in commercially available standard articles which may be joined together by standard means such as embossing (e.g. thermal bonding) or gluing or combination of both, and the articles may be produced industrially by conventional means.

### TEST METHODS

### Viability Test Method

Viability of microorganisms in patches comprising a dissolvable fibrous element comprising one or more microorganisms is determined as follows.

### Sample Preparation

Patches to be tested are removed from any protective packaging. Patches are tested neat without any protective packaging. The patches to be tested are conditioned at 25°C +/- 2°C and 60% +/- 2% relative humidity in an open container for 4 weeks and 8 weeks prior to testing and then tested immediately after 4 weeks or 8 weeks days of conditioning.

### Test Protocol

1. An entire patch or a part of a patch can be used as a sample. When a part of a patch is used as sample, a region of the patch where dissolvable fibrous elements comprising microorganisms are placed is identified, and cut and removed to include as much of the microorganism.
2. Weigh the sample.
3. Place the sample in a stomacher bag, and add an appropriate volume of a general purpose medium selected according to the microorganism included in the dissolvable fibrous element constituting the patch being tested to fully cover the sample. Take note the ratio of the weight of the sample to the volume of the medium for dilution and calculation as referenced in Step 9.
4. Stomach the stomacher bag containing sample for 5 min at 300 rpm and wait 15 min for foaming to reduce down to obtain a microorganism suspension.
5. Make serial dilutions of the microorganism suspension from Step 3 using 0.9% NaCl solution up to -8 (1:100000000).
6. Plate 100 µL of each of the serial dilutions from Step 4 onto a pre-poured petri plate selected according to the microorganism included in the dissolvable fibrous element constituting a patch being tested according to the standard spiral plating method known in the art in duplicate. If necessary, dry out and warm up the pre-poured petri plates , for example, by placing the plates in biosafety hood for about 30 min.
7. Invert each plate and incubate it under incubation conditions selected according to the microorganism included in the dissolvable fibrous element constituting the patch under aerobic conditions or anaerobic conditions in an anaerobe chamber or anaerobe box, depending on what microorganism being tested.
8. At the end of the incubation in Step 6, remove each plate and count colonies manually or using Q-Count from Spiral Biotech Spiral Biotech.
9. The total count (total level) of a microorganism present in the patch sample (CFU/patch) is calculated based on weight of the sample used and, the CFU count of the microorganism counted manually or obtained from the Q-Count software, and the dilution factor if the Q-Count software does not automatically factor in the dilution factor.

The log loss value is calculated as the difference between the final count of microorganisms and the initial (t=0day) count of microorganisms.

### Diameter Test Method

The average diameter of a discrete fibrous element or a fibrous element from a nonwoven or film is determined by using a Scanning Electron Microscope (SEM) or an Optical Microscope and an image analysis software. A magnification of 200 to 10,000 times is chosen such that the fibrous element is suitably enlarged for measurement. When using the SEM, the samples are sputtered with gold or a palladium compound to avoid electric charging and vibrations of the fibrous element in the electron beam. A manual procedure for determining the fibrous element diameters is used from the image (on monitor screen) taken with the SEM or the optical microscope. Using a mouse and a cursor tool, the edge of a randomly selected fibrous element is sought and then measured across its width (i.e., perpendicular to fibrous element direction at that point) to the other edge of the fibrous element. A scaled and calibrated image analysis tool provides the scaling to get actual reading in µm. For fibrous elements within a nonwoven or film, several fibrous elements are randomly selected across the sample of the nonwoven or film using the SEM or the optical microscope. At least two portions the nonwoven or film (or web inside a product) are cut and tested in this manner. Altogether at least 100 such measurements are made and then all data are recorded for statistical analysis. The recorded data are used to calculate average (mean) of the fibrous element diameters, standard deviation of the fibrous element diameters, and median of the fibrous element diameters.

### Dissolution Test Method for Filament

### Apparatus and Materials

Microscope: ViTiny VT300 Model VT-300 Digital Portable Microscope (manufactured by ViTiny USA) or equivalent;
25 mm x 75 mm microscope slides: Gold Seal® Products Rite-On® Micro Slides Catalog No. 3050, Gold Seal® Products, Portsmouth, N.H., or equivalent
22 mm x 22 mm microscope cover glass: Corning Labware Cover Glass No. 1 CS2000 Number 2845-22, or equivalent
Timer
De-ionized water (equilibrated at 23°C ± 1°C)
Tweezers
Test Protocol

Condition sealed filament samples at 21°C +/- 2°C and < 70% relative humidity for at least 2 hours prior to testing. If the filament samples are in the form of a bundle of filaments, use tweezers to pull off a small amount of the filaments (70-100 mg) from the bundle. Use a second tweezer to tease apart the small amount of filaments so that individual filaments are easily distinguished. Place the teased apart filaments on a microscope slide. Cover the teased apart filaments on the slide with a cover glass positioning it so that filaments are near the cover glass edge so that upon addition of water, quick and unimpeded water flow occurs over the filaments. The filaments are under a loading of about 3.4 x 10⁻⁴ g/mm² as a result of the cover glass being positioned on the filaments. Focus the microscope on the filaments near the cover glass edge. Begin a video recording of the filaments. Using a disposable transfer pipette, apply de-ionized water (200-250 mg) to the edge of the cover glass. Begin timer. When filaments are dissolved, end timer and record time of dissolution. The timing can be done by replaying the recorded video later. Three replicates of each filament sample are run and the average dissolution time is reported to within ± 5 seconds. Average dissolution time is in units of seconds.

### Dissolution Test Method for Web

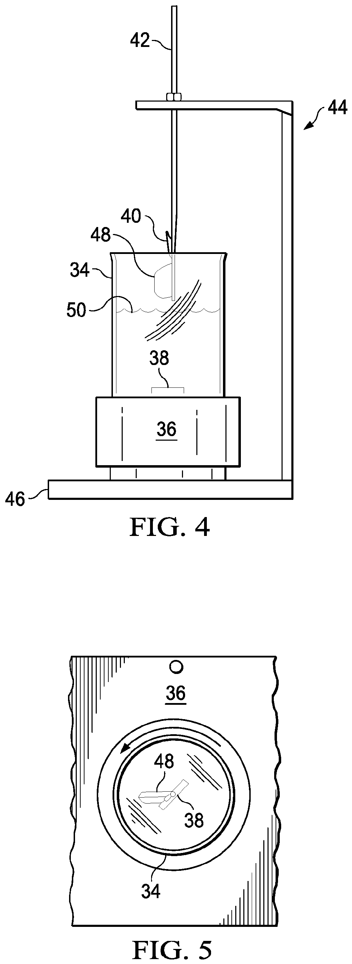

### Apparatus and Materials (also, see Figs. 4 and 5):

600 mL Beaker 34
Magnetic Stirrer 36 (Labline Model No. 1250 or equivalent)
Magnetic Stirring Rod 38 (5 cm)
Thermometer (1 to 100°C +/- 1 °C)
Cutting Die -- Stainless Steel cutting die with dimensions 3.8 cm x 3.2 cm
Timer (accurate to at least 0.1 second)
Alligator clamp (about one inch long) 40
Depth adjuster rod 42 and holder 44 with base 46
Polaroid 35 mm Slide Mount (commercially available from Polaroid Corporation or equivalent) and 35 mm Slide Mount Holder (or equivalent) 48
Deionized water (equilibrated at 23°C ± 1°C) 50

### Test Protocol

1. Equilibrate samples in constant temperature and humidity environment of 23°C ± 1 °C and 50%RH ± 2% for at least 2 hours.
2. Measure the basis weight of a sample to be tested using Basis Weight Test Method defined herein.
3. Cut at least three dissolution test specimens from the sample using cutting die (3.8 cm x 3.2 cm), so it fits within the 35 mm slide mount 48 which has an open area dimensions 24 x 36 mm.
4. Lock each specimen in a separate 35 mm slide mount 48.
5. Place magnetic stirring rod 38 into the 600 mL beaker 34.
6. Fill beaker 34 with 500 mL ± 5 mL of the deionized water 50.
7. Place full beaker 34 on magnetic stirrer 36, turn on stirrer 36, and adjust stir speed until a vortex develops and the bottom of the vortex is at the 400 mL mark on the beaker 34.

A trial run may be necessary to ensure the depth adjuster rod 42 is set up properly. Secure the 35 mm slide mount 48 in the alligator clamp 40 of the 35 mm slide mount holder such that the long end of the slide mount is parallel to the water surface. The alligator clamp 40 should be positioned in the middle of the long end of the slide mount. The alligator claim 40 is soldiered to the end of a depth adjuster rod 42. The depth adjuster rod 42 is set up in a way, so that when the slide mount 48 is lowered into the water, the entire specimen is completely submerged in the water at the center of the beaker 34, the top of the specimen is at the bottom of the vortex, and the bottom of the slide mount/slide mount holder is not in direct contact with the stirring rod 38. The depth adjuster rod 42 and alligator clamp 40 should be set so that the position of the specimen's surface is perpendicular to the flow of the water.

In one motion, drop the secured slide and clamp into the water and start the timer. The specimen is dropped so that the specimen is centered in the beaker. Disintegration occurs when the specimen breaks apart. Record this as the disintegration time. When all of the visible specimen is released from the slide mount, raise the slide out of the water while continuing the monitor the solution for undissolved specimen fragments. Dissolution occurs when all specimen fragments are no longer visible. Record this as the dissolution time. Three replicates of each sample are run and the average disintegration and dissolution times are recorded. Average disintegration and dissolution times are in units of seconds.

### In vitro Microorganism Transfer Test Method

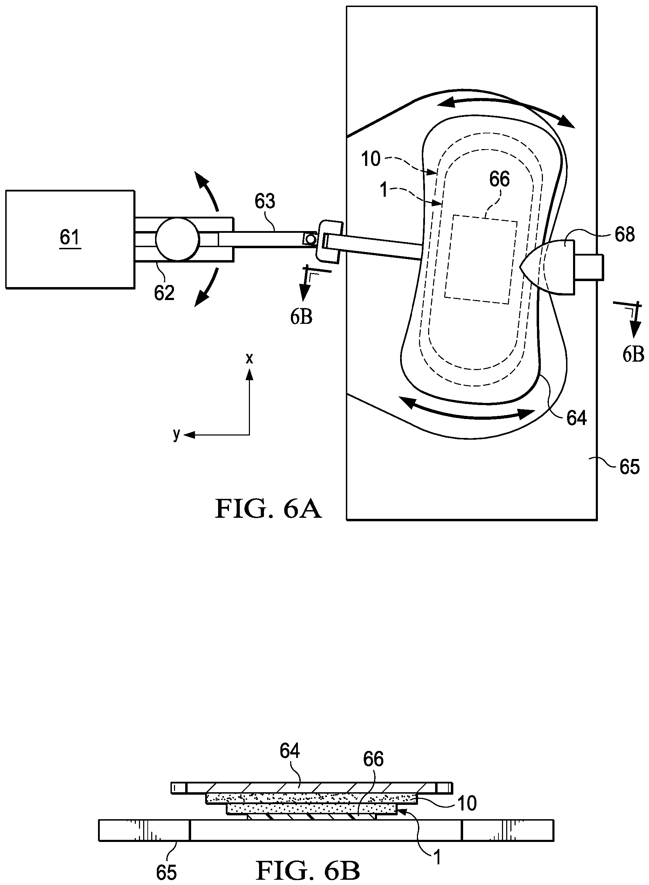

### Equipment (also, see Figs. 6A and 6B)

A rub tester (61) such as Southerland® 2000™ Rub Tester (Danilee Co., San Antonio, TX, USA) modified to have a connecting arm (63) connected to a moving U-shape arm (62) that moves in a horizontal motion (X-Y plane) is prepared. Connecting arm (63) is 14 cm long, with a pivot in the middle. At the other end, the U-shape arm (62) is connected to an aluminum sample holder (64) (19cm by 9cm, and weights 491g, via a connecting arm (63). The sample holder (64) sits on top of a flat silicone pad (65) such as AB-0129 (GARDCO, Pompano Beach, FL, USA). The rub tester (61) moves the U-shape arm (62) in a horizontal motion (X-Y plane) which then moves the connecting arm (63) in the same motion. Because there is a pivot in the middle of the connecting arm (63), the sample holder (64) has some freedom to move in a swivel pattern against the silicone surface. A fix piece (68) is secured against the sample holder (64), so the sample holder (64) can swivel from this point. A garment facing surface of a patch (1) is adhesive onto a body facing surface of an absorbent article (10) using an appropriate adhesive means such as an adhesive in the garment facing surface of the patch (1). The absorbent article is adhesive onto the surface of the sample holder (64) facing the silicone pad (65) using an appropriate adhesive means such as an adhesive in the backsheet of the absorbent article (10). In order to measure the amount of microorganism transferred, a film dressing (66) such as Tegaderm™ 1624W (3M, St. Paul, MN, USA) is secured with an adhesive tape onto a surface of the silicone pad (65). A size of the film dressing (66) used is preferably larger than an area of the patch (1) where dissolvable fibrous elements comprising microorganisms are included in order to secure as much as microorganisms in the patch (1) being transferred to the film dressing (66). The film dressing (66) is placed so that its center is in contact with the center of the patch (1) once in place. When the rub tester (61) is turned on, an area of the patch (1) containing the dissolvable fibrous element comprising microorganisms rubs against film dressing (66) swiveling. The distance traveled is about 2.2 cm horizontally (in the X direction) and about 1.1 cm vertically (in the Y direction). There needs to be a minimum distance traveled in order for the patch (1) to rub against a surface of the film dressing (66), and the distance traveled should be in such a way that the fibrous element in the patch (1) doesn't go out of the film dressing (66) while still rubbing against it. The distance traveled can be controlled by controlling a length of the connecting arm (63), a surface size and shape of the sample holder (64), and a location of the fix piece (68).

### Test Protocol

1. Place and secure the film dressing (66) onto a surface of the silicone pad (65). Equilibrate a patch (1) to be tested to room temperature for 15 minutes.
2. Secure the patch on top of an absorbent article by affixing a garment facing surface of the patch to a topsheet of the absorbent article.
3. Add an appropriate volume of sterile saline (0.9 % sodium chloride) to the center of the patch (1) to wet and dissolve the fibrous element, by letting the saline be absorbed for 3 minutes (+/-10 s). The volume of sterile saline can be adjusted depending on a type and/or size of patch. For example, 0.5 ml - 2 ml of sterile saline may be used for common sizes of sanitary napkins.
4. Secure the absorbent article (10) with the patch (1) to the sample holder (64) so that the backside of the absorbent article (10) adheres on the surface of the sample holder (64) using an appropriate means, and the center of a body facing surface of the patch (1) is in contact with the center of the film dressing (66).
5. Run the rub tester (61) for 5 minutes at a speed of 21 cycles per minute.
6. Lift the sample holder (64). Remove the film dressing (66) and place it in a bottom of a 100 mm sterile, plastic petri dish, so that a backside of the film dressing (66) adheres to the bottom of the petri dish. If the dressing is too big for the 100 mm dish, it can be cut in sections to fit, or a larger dish is used.
7. Add 10 mL of a general purpose medium selected according to the microorganism included in the patch to the 100mm petri dish. If a larger dish is used, add the appropriate amount of media for the dish size to cover the dressing. The Medium can contain low concentrations of non-ionic surfactant such as Tween and Lecithin which can aid in the removal of the microorganism from the film dressing.
8. Swirl the petri dish on an orbital shaker at 100 rpm for 20 min at 33°C to obtain to a microorganism suspension.
9. Make serial dilutions of the microorganism suspension from step 7 using 0.9% NaCl (saline) up to 10⁻⁸ (1:100000000).
10. Plate 100 µL of each of the serial dilutions from Step 8 onto a pre-poured petri plate selected according to the microorganism included in the patch being tested according to the standard spiral plating method known in the art in triplicate. Invert and incubate each plate under incubation conditions selected according to the microorganism under aerobic conditions or anaerobic conditions in an anaerobe chamber or anaerobe box, depending on what microorganism being tested.
11. After removing each plate, count colonies in each plate manually or using Q-Count from Spiral Biotech Spiral Biotech. Calculate the total count (total level) of microorganism (CFU/article) transferred based on the CFU count of the microorganism counted manually or obtained from the Q-Count software, and a dilution factor if the Q-Count software does not automatically factor.

### EXAMPLES

### Examples 1-3. Filament preparation

Examples 1-3 are non-limiting examples of a filament-forming composition for fibrous elements according to the present invention.

**Table 1**

| | Example 1 | Example 2 | Example 3 |
|---|---|---|---|
| Ingredients of Filament Forming Composition | grams | Grams | Grams |
| Propyl gallate¹ (antioxidant) | 0.06 | 0.06 | 0.056 |
| Trehalose² (stabilizing agent) | 4.29 | 4.29 | 4.29 |
| Sodium Citrate Dehydrate³ (pH buffering agent) | 0.15 | 0.15 | 0.155 |
| ⁴Sodium Caseinate⁴ (stabilizing agent) | 1.53 | 1.53 | 1.54 |
| Distilled Water | 54.53 | 54.53 | 42.45 |
| *L. fermentum* 297R1 | 1.86 | | 1.85 |
| *B infantis* 35624 | | 1.86 | |
| Polyvinyl alcohol ⁵ (filament-forming material) | 10.88 | 10.88 | 10.88 |

| | | | |
|---|---|---|---|
| ¹Propyl gallate (Spectrum Chemicals, Gardena, CA) ²Trehalose (Swanson Ultra, Fargo, ND) ³Sodium Citrate Dehydrate (Sigma Aldrich, St. Louis, MO) ⁴Sodium Caseinate (Sigma Aldrich, St. Louis, MO) ⁵Polyvinyl alcohol (Sekisui Specialty Chemical Company, Dallas, TX) | | | |

The filament-forming compositions of Examples 1 and 2 were prepared as follows.

First, 23% polyvinyl alcohol solution was prepared. A wide mouth pint jar in a water bath with over head stirrer fitted through a holed lid and a stir blade nearly as wide as the jar was set up. 231g distilled water was added to the jar. With moderate stirring, 69g polyvinyl alcohol was slowly added. Water bath was turned on heated to 70°C. The water bath was turned off when all of the polyvinyl alcohol was dissolved, and was allowed to cool to 50°C. The jar was removed from the stirrer, and sit sealed while cooling to 23°C. All of the air bubbles were removed as it sat.

Second, a stock cryoprotecting solution (25% solids) having a composition as below was prepared.

**Table 2**

| Ingredients | % in stock cryoprotection solution | Amount (g) for a 200g batch |
|---|---|---|
| Propyl gallate | 0.23% | 0.46 |
| Trehalose | 17.77% | 35.54 |
| Sodium citrate dihydrate³ | 0.64% | 1.28 |
| Sodium caseinate | 6.36% | 12.72 |
| Distilled water | 75% | 150 |

All ingredients except sodium caseinate were dissolved in 75mL distilled water by heating to 60°C with stirring to form a trehalose solution, and the obtained trehalose solution was cool down to room temperature. Sodium caseinate was dispersed in 75mL distilled water and heat to 60°C to form a caseinate solution. Obtained caseinate solution was autoclaved at 121°C for 60 minutes and then cooled to 45°C. The trehalose solution was added into the caseinate solution. The solution was brought to total weight of 200g by rinsing trehalose solution jar with distilled water and adding the distilled water. Obtained stock cryoprotecting solution was seal and stored in refrigerator.

Next, a filament-forming composition was obtained as follows:
1. 27.3g of the cool cryoprotectant solution and 1.86g microorganisms were mixed for 4 minutes at 3500 rpm using a SpeedMixer or an equivalent.
2. 26g of the resulting mixture from step 1 were added into 47.3g of the polyvinyl alcohol solution from above and mixed for 4 min at 3500 rpm using a SpeedMixer or an equivalent.
3. The resulting solution was transferred to a filament spinning apparatus as shown in FIG. 2, and filaments were produced according to the method as described in METHOD FOR PRODUCTION and filament spinning settings below.

The filament-forming compositions of Example 3 was prepared as follows.

First, 19% polyvinyl alcohol solution was prepared. A wide mouth pint jar in a water bath with over head stirrer fitted through a holed lid and a stir blade nearly as wide as the jar was set up. 243g distilled water was added to the jar. With moderate stirring, 57g polyvinyl alcohol was slowly added. Water bath was turned on heated to 70°C. The water bath was turned off when all of the polyvinyl alcohol was dissolved, and was allowed to cool to 50°C. The jar was removed from the stirrer, and sit sealed while cooling to 23°C. All of the air bubbles were removed as it sat.

Second, a stock cryoprotecting solution (25% solids) having a composition as below was prepared.

**Table 3**

| Ingredients | % in stock cryoprotection solution | Amount (g) for a 200g batch |
|---|---|---|
| Propyl gallate | 0.23% | 0.46 |
| Trehalose | 17.77% | 35.54 |
| Sodium citrate dihydrate³ | 0.64% | 1.28 |
| Sodium caseinate | 6.36% | 12.72 |
| Distilled water | 75% | 150 |

All ingredients except sodium caseinate were dissolved in 75mL distilled water by heating to 60°C with stirring to form a trehalose solution, and the obtained trehalose solution was cool down to room temperature. Sodium caseinate was dispersed in 75mL distilled water and heat to 60°C to form a caseinate solution. Obtained caseinate solution was autoclaved at 121°C for 60 minutes and then cooled to 45°C. The trehalose solution was added into the caseinate solution. The solution was brought to total weight of 200g by rinsing trehalose solution jar with distilled water and adding the distilled water. Obtained stock cryoprotecting solution was seal and stored in refrigerator.

Next, a filament-forming composition was obtained as follows:
1. 18.2g of the cool cryoprotectant solution and 1.4g microorganisms were mixed for 4 minutes at 3500 rpm using a SpeedMixer or an equivalent.
2. 17.3g of the resulting mixture from step 1 were added into 37.54g of the polyvinyl alcohol solution from above and mixed for 4 min at 3500 rpm using a SpeedMixer or an equivalent.
3. The resulting solution was transferred to a filament spinning apparatus as shown in FIG. 2, and filaments were produced according to the method as described in METHOD FOR

### PRODUCTION.

Diameters of filaments of Example 3 were measured according to Diameter Test Method described in METHOD FOR PRODUCTION section. SEM images of 7 portions of the fibers were obtained, and 20 diameters from multiple filaments per each portion were manually measured. An average diameter of filaments of Example 3 was 20.85um with a standard deviation of 7.723um. A median was 19.43um.

### Example 4: Nonwoven web preparation

A nonwoven web was formed from filaments of Example 3 in Table 1 by collecting the filaments on a collection device, such as a belt or fabric.

### Example 5: Pantiliner

As a sample of patchs, a pantiliner containing a nonwoven patch from the web prepared in Example 4 was prepared using Always Incredible Thin Liner currently sold by The Procter & Gamble Company. The liner was open, and frozen in a corner using a CytoFreeze spray. The topsheet was carefully separated and removed from the other part of the liner. Nonwoven (14 gsm Bico 70/30 Phillic Nonwoven, Pegas, Czech Republic) was cut into the same size and shape as the removed topsheet. Glue (H2031 C5X Hot Melt Adhesive- 0.009g/in², Henkel, Germany) was applied onto an upper surface of the liner where a core of the liner was exposed. The web prepared in example 4 was cut into a rectangular (100mg, 62mm x 21mm) patch, and applied on the center of the glued core. The cut Pegas nonwoven was placed on the upper surface of the liner to cover the entire upper surface of the liner, and pressed to get the nonwoven glued to the liner. The article was frozen until being placed in tests.

### Example 6: Patch

Nonwoven (14 gsm Bico 70/30 Phillic Nonwoven, Pegas, Czech Republic) was cut to 75 mm x 30 mm to prepare a first nonwoven layer. Glue (H2031 C5X Hot Melt Adhesive- 0.005g/in², Henkel, Germany) was applied to one surface (body facing surface) of the first nonwoven. A nonwoven web prepared in Example 4 was cut into a rectangular (100mg, 62mm x 21mm) shape, and placed on the center of the body facing surface of the first nonwoven where the glue was applied. The same nonwoven as the first nonwoven layer was cut to the same size as the first nonwoven layer to prepare a second nonwoven layer. The second nonwoven layer was placed on top of the web such that the second nonwoven layer covered the entire web and the first nonwoven layer, and pressed to get the first and second nonwoven layers and the web glued together. Two 12 mm glue strips were applied to the other surface (garment facing surface) of the first nonwoven layer in a longitudinal direction of the nonwoven layer. A release paper (??) was disposed on to the garment facing surface of the first nonwoven layer where the two strip glue were applied to cover the entire garment facing surface. The patch was frozen until being placed in tests.

### Comparative example 1: Pantiliner

As a comparison sample, pantiliners containing lyophilized *L. fermentum* powder instead of the nonwoven patch from the web prepared in example 4 was prepared using Always Incredible Thin Liner currently sold by The Procter & Gamble Company. The liner was open, and frozen in a corner using a CytoFreeze spray. The topsheet was carefully separated and removed from the other part of the liner. Nonwoven (14 gsm Bico 70/30 Phillic Nonwoven, Pegas) was cut into the same size and shape as the removed topsheet. Glue (H2031 C5X Hot Melt Adhesive- 0.009g/in², Henkel) was applied onto an upper surface of the liner where a core of the liner was exposed. 10 mg of lyophilized *L. fermentum* powder were applied on the center of the glued core. The cut Pegas nonwoven was placed on the upper surface of the liner to cover the entire upper surface of the liner, and pressed to get the nonwoven glued to the liner. The article was frozen until being placed in tests.

### Example 7: Dissolution Test

Dissolution of filaments of Example 3, and a web prepared in Example 4 were conducted and dissolution times were measured according to Dissolution Test Method for Filament and Dissolution Test Method for Web described in METHOD FOR PRODUCTION section. For filaments, 5 times dissolution tests were conducted, and an average dissolution time was about 8 seconds with a standard deviation of 2 seconds. For web, 4 samples were cut from the web prepared in Example 4 and used for dissolution test. An average dissolution time was about 10 minutes and 27 seconds with a standard deviation of 76 seconds.

### Example 8: Viability Test

Viabilities of microorganisms using pantiliners of Example 5 and Comparative example 1 were measured and log loss values after 4 and 8 week incubation were determined according to Viability Test Method described in METHOD FOR PRODUCTION section. MRSA (DeMan, Rogosa and Sharpe Agar) plates and MLBT (Modified Letheen Broth + Tween) media (Difco) were used as pre-poured petri plates and a general purpose medium, respectively. After plating plate 100 µL of serial dilutions on MRSA plates, the plates were inculated for 48 hour at 33°C under aerobic conditions in an aerobe chamber or aerobe box.

Results are summarized in Table 4.

**Table 4**

| Incubation at 25°C / 65 % RH | Initial Log average CFU/ article @ t = 0 wks | Log average CFU/article @ t = 4 wk | Log average CFU/article @ t = 8 wk | Δ Log for 4 wks (t=0 - t=4wks) | Δ Log for 8 wks (t=0 - t=8wks) |
|---|---|---|---|---|---|
| Pantiliner of Example 5 | 7.08 | 6.97 | 6.11 | 0.11 | 0.97 |
| Pantiliner of Com. example 1 | 8.92 | 5.81 | 3.93 | 3.11 | 5.81 |

### Example 9: Transfer of Microorganism Test

Transfer of microorganisms was measured using the patch of Example 6 according to In vitro Microorganism Transfer Test Method described in METHOD FOR PRODUCTION section. Results are summarized in Table 5.

**Table 5**

| Amount of saline added to a patch | Initial log CFU in a patch (CFU/article) | Log CFU transferred from a patch (CFU/article) |
|---|---|---|
| 0.5 ml | 5.5 | 4.3 |
| 2 ml | 5.5 | 4.1 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 µm" is intended to mean "about 40 µm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a reference, the meaning or definition assigned to that term in this document shall govern.

## Claims

1. A patch comprising a dissolvable fibrous element comprising a filament-forming material and a microorganism, further comprising
a liquid pervious first layer comprising a body facing surface and a garment facing surface opposite to the body facing surface,
an adhesive disposed at least partially on the garment facing surface of the first layer, and
a removable release liner at least partially covering the adhesive.

2. The patch according to claim 1, wherein the dissolvable fibrous element comprises a spun filament comprising a microorganism.

3. The patch according to claim 1 or 2, wherein the first layer comprises the dissolvable fibrous element.

4. The patch according to any of the preceding claims further comprising a liquid pervious second layer disposed onto the body facing surface of the first layer wherein the second layer comprises a body facing surface.

5. The patch according to claim 4, wherein the patch further comprises a liquid pervious third layer disposed onto the body facing surface of the second layer.

6. The patch according to claim 4 or 5, wherein the second layer comprises the dissolvable fibrous element.

7. The patch according to claim 5, wherein the third layer comprises the dissolvable fibrous element.

8. The patch according to claim 4, wherein the dissolvable fibrous element is disposed between the first layer and the second layer in a form of fibers.

9. The patch according to any of the preceding claims, wherein the adhesive is liquid pervious.

10. The patch according to any of the preceding claims, wherein the microorganism is a probiotic.

11. The patch according to any of the preceding claims, wherein the dissolvable fibrous element exhibits an average diameter of greater than 1µm as measured according to the Diameter Test Method.

12. The patch according to any of the preceding claims, wherein the patch further comprises at least one agent selected from the group consisting of a prebiotic, an organic acid, a skin care active, a stabilizing agent, an antioxidant, a plasticizer, a colorant, TSST-1 reducing material, and mixtures thereof.

13. The patch according to any of the preceding claims, wherein the dissolvable fibrous element further comprises at least one agent selected from the group consisting of a prebiotic, an organic acid, a skin care active, a stabilizing agent, an antioxidant, a plasticizer, a colorant, TSST-1 reducing material, and mixtures thereof.

14. A kit comprising at least one patch according to any of the preceding claims and at least one absorbent article.

## Patentansprüche

1. Patch, umfassend ein lösliches faserhaltiges Element, das ein fadenbildendes Material und einen Mikroorganismus umfasst, ferner umfassend
eine flüssigkeitsdurchlässige erste Schicht, umfassend eine körperseitige Oberfläche und eine bekleidungsseitige Oberfläche gegenüber der körperseitigen Oberfläche,
ein Haftmittel, das wenigstens teilweise auf der bekleidungsseitigen Oberfläche der ersten Schicht angeordnet ist, und
eine entfernbare Abziehfolie, die das Haftmittel wenigstens teilweise bedeckt.

2. Patch nach Anspruch 1, wobei das lösliche faserhaltige Element einen gesponnenen Faden umfasst, der einen Mikroorganismus umfasst.

3. Patch nach Anspruch 1 oder 2, wobei die erste Schicht das lösliche faserhaltige Element umfasst.

4. Patch nach einem der vorstehenden Ansprüche, ferner umfassend eine flüssigkeitsdurchlässige zweite Schicht, die auf der körperseitigen Oberfläche der ersten Schicht angeordnet ist, wobei die zweite Schicht eine körperseitige Oberfläche umfasst.

5. Patch nach Anspruch 4, wobei das Patch ferner eine flüssigkeitsdurchlässige dritte Schicht umfasst, die auf der körperseitigen Oberfläche der zweiten Schicht angeordnet ist.

6. Patch nach Anspruch 4 oder 5, wobei die zweite Schicht das lösliche faserhaltige Element umfasst.

7. Patch nach Anspruch 5, wobei die dritte Schicht das lösliche faserhaltige Element umfasst.

8. Patch nach Anspruch 4, wobei das lösliche faserhaltige Element zwischen der ersten Schicht und der zweiten Schicht in einer Form von Fasern angeordnet ist.

9. Patch nach einem der vorstehenden Ansprüche, wobei das Haftmittel flüssigkeitsdurchlässig ist.

10. Patch nach einem der vorstehenden Ansprüche, wobei der Mikroorganismus ein Probiotikum ist.

11. Patch nach einem der vorstehenden Ansprüche, wobei das lösliche faserhaltige Element einen durchschnittlichen Durchmesser von über 1 µm gemäß Messung nach dem Durchmesserprüfverfahren aufweist.

12. Patch nach einem der vorstehenden Ansprüche, wobei das Patch ferner wenigstens ein Mittel umfasst, das ausgewählt ist aus der Gruppe bestehend aus einem Präbiotikum, einer organischen Säure, einem Hautpflegewirkstoff, einem Stabilisierungsmittel, einem Antioxidationsmittel, einem Weichmacher, einem Farbmittel, einem TSST-1 reduzierenden Material und Mischungen davon.

13. Patch nach einem der vorstehenden Ansprüche, wobei das lösliche faserhaltige Element ferner wenigstens ein Mittel umfasst, das ausgewählt ist aus der Gruppe bestehend aus einem Präbiotikum, einer organischen Säure, einem Hautpflegewirkstoff, einem Stabilisierungsmittel, einem Antioxidationsmittel, einem Weichmacher, einem Farbmittel, einem TSST-1 reduzierenden Material und Mischungen davon.

14. Satz, umfassend wenigstens ein Patch nach einem der vorstehenden Ansprüche und wenigstens einen Absorptionsartikel.

## Revendications

1. Patch comprenant un élément fibreux soluble comprenant un matériau de formation de filaments et un micro-organisme, comprenant en outre
une première couche perméable aux liquides comprenant une surface tournée vers le corps et une surface tournée vers le vêtement opposée à la surface tournée vers le corps,
un adhésif disposé au moins partiellement sur la surface tournée vers le vêtement de la première couche, et
une protection détachable amovible recouvrant au moins partiellement l'adhésif.

2. Patch selon la revendication 1, dans lequel l'élément fibreux soluble comprend un filament filé comprenant un micro-organisme.

3. Patch selon la revendication 1 ou 2, dans lequel la première couche comprend l'élément fibreux soluble.

4. Patch selon l'une quelconque des revendications précédentes comprenant en outre une deuxième couche perméable aux liquides disposée sur la surface tournée vers le corps de la première couche dans lequel la deuxième couche comprend une surface tournée vers le corps.

5. Patch selon la revendication 4, dans lequel le patch comprend en outre une troisième couche perméable aux liquides disposée sur la surface tournée vers le corps de la deuxième couche.

6. Patch selon la revendication 4 ou 5, dans lequel la deuxième couche comprend l'élément fibreux soluble.

7. Patch selon la revendication 5, dans lequel la troisième couche comprend l'élément fibreux soluble.

8. Patch selon la revendication 4, dans lequel l'élément fibreux soluble est disposé entre la première couche et la deuxième couche sous la forme de fibres.

9. Patch selon l'une quelconque des revendications précédentes, dans lequel l'adhésif est perméable aux liquides.

10. Patch selon l'une quelconque des revendications précédentes, dans lequel le micro-organisme est un probiotique.

11. Patch selon l'une quelconque des revendications précédentes, dans lequel l'élément fibreux soluble présente un diamètre moyen supérieur à 1 µm tel que mesuré selon le procédé de test de diamètre.

12. Patch selon l'une quelconque des revendications précédentes, dans lequel le patch comprend en outre au moins un agent choisi dans le groupe constitué d'un prébiotique, d'un acide organique, d'un agent actif pour le soin de la peau, d'un agent stabilisant, d'un antioxydant, d'un plastifiant, d'un colorant, d'un matériau réducteur de TSST-1, et de mélanges de ceux-ci.

13. Patch selon l'une quelconque des revendications précédentes, dans lequel l'élément fibreux soluble comprend en outre au moins un agent choisi dans le groupe constitué d'un prébiotique, d'un acide organique, d'un agent actif pour le soin de la peau, d'un agent stabilisant, d'un antioxydant, d'un plastifiant, d'un colorant, d'un matériau réducteur de TSST-1, et de mélanges de ceux-ci.

14. Trousse comprenant au moins un patch selon l'une quelconque des revendications précédentes et au moins un article absorbant.
